# EUROPEAN PATENT APPLICATION

(11) **EP 1 842 915 A1**
(43) Date of publication of application: **10.10.2007**
(21) Application number: 06290534.4
(22) Date of filing: 04.04.2006
(51) Int. Cl.: C12N 15/10

(54) **Method of in vitro polynucleotide sequences shuffling by recursive circular DNA molecules fragmentation and ligation**

(71) Applicant: Libragen, 31000 Toulouse (FR)
(72) Inventor: Lefevre, Fabrice, 32120 Bajonnette (FR); Nalin, Renaud, 31650 Auzielle (FR); Jarrin, Cyrille, 31600 Seysses (FR)
(74) Representative: Gallois, Valérie

(57) **Abstract**

The present invention relates to a method for *in vitro* recombining polynucleotides sequences of interest from at least two initial different polynucleotide sequences of interest by cleavage/ligation of circular DNA molecules.

## Description

### FIELD OF THE INVENTION

The present invention relates to the field of molecular biology and, more parlticularly to the *in vitro* evolution.

### BACKGROUND OF THE INVENTION

Natural biodiversity is a huge reservoir of protein scaffolds that can be exploited for industrial applications. By screening cultivable micro-organisms, an impressive number of enzymes have been discovered and applied to industrial processes including but not limited to the fields of feed and food processing (xylanases, phytases, amylases, glucose isomerases, laccases, pectinases, chitinases,...), textile treatment (amylases, lipases, xylanases,...), paper production (xylanases, cellulases,...), fine chemistry and pharmaceutical applications (alcohol oxidases, transaminases, epoxide hydrolases, ketone reductases, amidases, esterases, lipases, peroxydases...), cosmetics (glycosylases, glycosyl-transferases, keratinases), environment (dehalogenases, monoxygenases, ...). The recent development of improved molecular biology tools has enabled to mine for enzymes from non-cultivatable bacteria through the metagenomic approach (U.S. Patent #6,989,249 ; Handlesman, 2004, Microb. Mol. Biol. Rev., 68 (4), 669-685 ; Cottrell et al., 1999 , Appl. Environ. Microbiol., 65, 2553-2557; Entcheva et al., 2001 , Appl. Environ. Microbiol. 67, 88-89; Henne et al., 1999, Appl. Environ. Microbiol., 65, 3901-3907; Henne et al., 2000, Appl. Environ. Microbiol., 66, 3113-3116; Knietsch et al., 2003a , Appl. Environ. Microbiol., 69, 1408-1416; Knietsch et al., 2003b, Appl. Environ. Microbiol., 69, 3048-3060; Knietsch et al., 2003c, J. Mol. MicrobioL Biotechnol., 5, 46-56; Lee et al., 2004, Appl. Microbiol. Biotechnol., 65, 720-726; Majernik et al., 2001, J. Bacteriol.,183, 6645-6653; Rondon et al., 2000, Appl. Environ. Microbiol., 66, 2541-2547; Voget et al., 2003, Appl. Environ. Microbiol., 69, 6235-6242; Yun et al., 2004, Appl. Environ. Microbiol., 70, 7229-7235). This novel technology enables to provide new enzyme activities that would have not be obtained previously as the parent micro-organisms can not been cultivated or even isolated with usual microbiology methods.

If biocatalysis is now routinely used at the industrial scale, the increasing economic pressure for better yields, higher product quality, lower by-products generation, and increased biocatalyst stability requires to finely tune natural enzymes to their final industrial application. Such an adaptation may be for example a better thermo, acido or alkalo-stability, a better product toxicity resistance, a higher turn-over at high substrate concentration, an increased enantiospecificity, or a new regioselectivity. This adaptation can be achieved through steps of protein engineering. Several methods have been used and described so far.

These methods to create new improved protein/enzyme variants can be classified in three categories:
- site directed mutagenesis methods,
- random mutagenesis methods,
- recombination methods.

The principle of site directed mutagenesis or "rational design" is the selective replacement of amino acids to improve proteins performances. It is based on intensive studies of the target protein, including the resolution of the three-dimensional structure, and the comprehension of the mechanisms which are subjacent to the acquisition of a particular property. For example, an increased thermostability can be acquired by the introduction of disulphide bridges or by a reinforcement of the hydrophobic network within the protein (Wakarchuk et al., 1994, Prot. Eng., 7, 1379-1386). Developed in the years 1980, rational design was a real success, like the adaptation of proteases or lipases for detergents applications (Svendsen et al.,1995, Biochem. Biophys. Acta, 1259, 9-17 ; Aehle et al., 1993, J. Biotechnol., 28, 31-40). Nevertheless, the rate of failure of this approach is important and the improvements are restricted with simple modifications. Indeed, the relations structure/function are difficult to apprehend and the improvements are rarely predictable. Moreover, the structural data necessary to the rational design are not always immediately available and the acquisition of a new structure remains a heavy investment of time and work.

Random mutagenesis methods generate new sequences by specific changes of the initial sequence of a gene. Various methods of random mutagenesis were described : by means of UV treatment of the polynucleotide sequences, by chemical agents treatment, by use of strains naturally introducing mutations or by error prone PCR amplification. The latter is the most method used for *in vitro* random mutagenesis, as it is certainly the simplest to implement and to control. It is based on the capacity of thermostable polymerases to spontaneously introduce mutations during the duplication of the genetic material. The absence of 3' -5 ' "proof-reading" exonucleasic activity of Taq DNA polymerase, leads to mutation rates of about 0,01% (1 change for 10000 Pb) in conditions of optimal reaction, which is a rate ten times higher than that of polymerases presenting a "proof-reading" activity like Pfu DNA polymerase (Cline et al. , 1996, Nucleic Acids Res., 24(18), 3546-51). A rate of mutation of 0,01% is however insufficient for an effective random mutagenesis, the majority of the generated products having the wild type sequences. The fidelity of polymerases such as Taq DNA polymerase is then lowered by using unbalanced concentrations of dNTP and by increasing ionic concentrations in the reaction medium until sufficient important mutation rates (0,1 to 1-2%) are obtained to generate one to several tens of changes per target gene (Cirino et al., 2003, Methods Mol. Biol., 231, 3-9).

However, the error prone PCR is a strongly biased random method as certain types of changes are favoured compared to others (Cadwell and Joyce, 1992, PCR Methods Appl., 1, 28-33 ; Vartanian et al., 1996, Nucleic Acids Res., 24, 2627-2631). This bias leads to mutants libraries of low quality where certain positions of the gene and certain changes are over represented with the detriment of other possible beneficial mutations, which represents a major limit of the method to obtain improved protein/enzymes variants.

Moreover, all the random mutagenesis methods of polynucleotide sequences suffer from a skew in the codon change. Due to the genetic code, the change of a particular codon in another is more or less facilitated (the change of a serine in threonine is statistically more probable than that of an histidine to a tryptophan which requires three changes on the same codon).

Codon random mutagenesis methods were developed to allow the analyze of amino acid changes that are impossible to obtain by usual random mutagenesis of nucleic acids. In such methods, each codon of a gene is specifically replaced by another codon using for example PCR and a set of primers bearing random triplets representative of the 20 possible amino acids. The whole gene is then rebuilt by PCR with overlapping primers to obtain a gene library. Though obviously simple, such methods are however expensive and very time consuming.

On the opposite, molecular recombination methods do not generate additional mutations but exploits a pre-existing sequence diversity to rearrange these sequences in a new original way. *The in vitro* recombination aims to create new sequences potentially encoding for improved proteins/enzymes. In term of evolution, the recombination makes it possible to very quickly cumulate beneficial changes for the required improvement and to eliminate the harmful changes (Harayama, 1999, TIBTECH, 16, 76-82 ; Tobin et al., 2000, Curr. Opin. Struct. Biol., 10, 421-427).

The existing methods *for in vitro* recombination of polynucleotidic sequences are all based on homologous DNA fragments exchange during cycle(s) of denaturation/hybridization. This DNA fragments exchange makes it thus possible to shuffle the genetic information carried by various parental sequences.

One of the first methods of *in vitro* recombination was the DNA Shuffling invented by WPC Stemmer (Stemmer, 1994, Nature, 370, 389-391 ; Stemmer, 1994, Proc. Natl. Acad. USA, 91, 10747-10751 ; Patent US6132970). In this method, parental sequences are digested by DNAse I. The fragments are then used to rebuild whole genes thanks to a PCR step carried out without primers.

The StEP method (Staggered Extension Process), developed later after, distinguished from DNA Shuffling by the absence of DNAse I digestion (Zhao et al., 1998, Nature Biotech., 16, 258-261). The information carried by the parental sequences is rearranged thanks to an incomplete polymerization by using very short elongation times during PCR.

These two techniques of molecular recombination present several disadvantages :
- first, the efficiency of recombination is strongly dependent on the homology level between the parental sequences. The less homologous the parental genes will be, the less recombination events will occur. With low homology genes, a contamination of the recombined population of genes occurs by the parental sequences, that are favoured during the hybridization step (self hybridization is more rapid than heterologous one).
- second, the recombination process used relies on polymerases, so that additional unwanted mutations are introduced (indirect effect of the fidelity of the polymerases), which decreases the chances to obtain recombined genes encoding for improved proteins.

Various technical alternatives *for in vitro* DNA recombination were developed to face these problems.

For *in vitro* recombination of two genes showing a low level of homology, preparation of single strand DNA fragments has been tested (one of this single strand approach was the RACHITT method (RAndom CHImeragenesis one Transient Templates - Coco et al., 2001, Nature Biotech., 19, 354-359). One strand is for example the coding strand of one of the parental genes, and the other stands are the non-coding strands of the other parental genes. This allows to force the recombination and to limit the rebuilding of parental genes. Such a method has been explored to recombine two parental genes coding for two enzymes having 84% of identity in amino acids, and resulted in an increase of the recombination efficiency from 1% (using traditional DNA shuffling) to up to 14% using single strand DNA Shuffling (Kikuchi et al., 2000, Gene, 243, 133-137). Though improved, the single stranded recombination methods are more time and effort consuming. Their efficiency remains rather low, and they are not suited for an efficient statistical generation of polynucleotide sequences encoding improved protein variants.

The problem of polymerase fidelity was a well known drawback of the DNA Shuffling method, as the initial method of DNA-Shuffling was reported by Stemmer to show a spontaneous mutation rate of 0,7%, comparable with that obtained by PCR error prone Stemmer (Stemmer, 1994, Nature, 370, 389-391). Modified protocols have been developed to decrease this unwanted mutation rate by using proof reading polymerases instead of Taq DNA polymerase. Zhao and Arnold (Zhao and Arnold, 1997, Nucleic Acids Res., 25, 1307-1308) have shown that very specific conditions (called high-fidelity DNA shuffling), that are far away from the original conditions described by Stemmer, only lead from 46% to 95% of active clones whereas 100% should be expected. So even under these conditions, some mutations are still introduced by the polymerase during recombination, thus lowering the chances to discover some improved mutants, and increasing the overall population of clones to be screened.

Another method was developed in 1998 to avoid the use of polymerases, source of mutations, by employing a ligation chain reaction based method (LCR, Landegren et al., 1988, Science, 241, 1077-1080). This method called L-Shuffling (Patent WO 0009679) was based on the denaturation/hydridation of fragments of DNA which are then assembled by a thermostable ligase. As for DNA-shuffling, this method relies on the homology level of DNA fragments, and on hybridization events so that its efficiency can slow down as the homology decreases.

### SUMMARY OF THE INVENTION

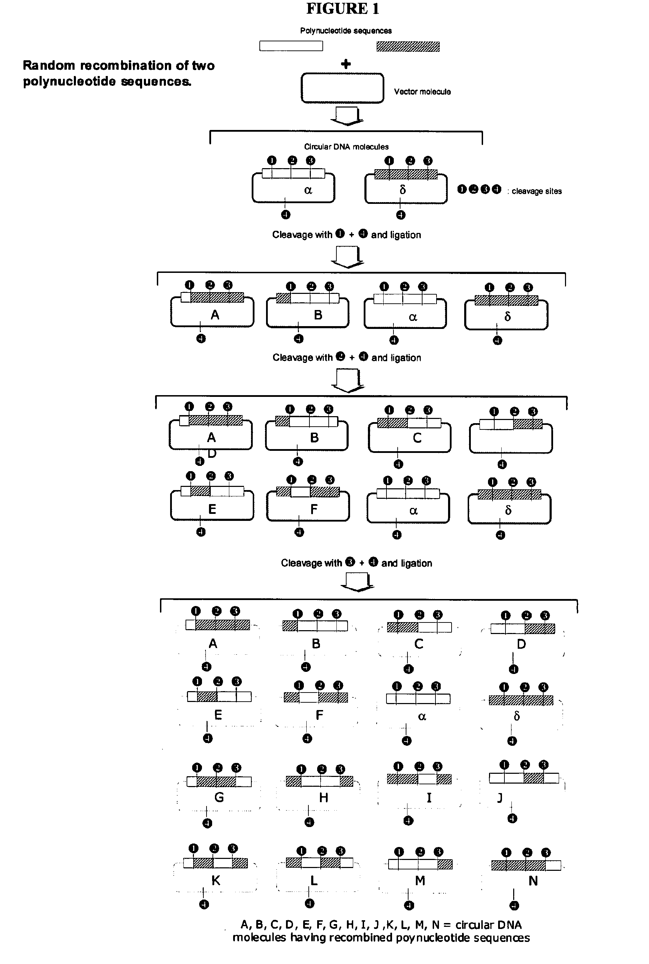

To solve the different problems encountered in the field of directed evolution of polynucleotide sequences, the present invention aims at providing an *in vitro* recombination method of polynucleotide sequences of interest that does not rely on DNA fragment hybridization, nor on DNA polymerization. In a method of this invention, each polynucleotide sequences of interest is cloned in a vector molecule, to obtain a circular DNA molecule. A cleavage of one circular DNA molecule is achieved at a unique location of the vector molecule, and at one or several locations of the polynucleotide sequence of interest, thus generating at least two linear fragments. These fragments are mixed with linear fragments derived from the cleavage of another circular DNA molecule obtained from another polynucleotide sequence of interest. A ligase is then used to randomly reassemble the linear fragments into new circular DNA molecules (see Figure 1).

As the vector molecule has been cleaved at a single site, ligase reassembles the two ends of the vector molecule, while randomly ligating the end of one fragment of a polynucleotide sequence of interest with the end of another fragment of a polynucleotide sequence of interest, thus recombining these polynucleotide sequences of interest.

The process of cleaving circular DNA molecule at other cleavage sites of the polynucleotide sequences of interest, mixing the resulting linear fragments and ligating them to randomly reassemble circular DNA fragments can be repeated to further recombine the polynucleotide sequences of interest.

Therefore, the present invention concerns a method *for in vitro* recombining polynucleotides sequences of interest from at least two initial different polynucleotide sequences of interest by cleavage/ligation of circular DNA molecules, comprising :
1) providing at least two circular DNA molecules, each comprising a vector molecule and one of the at least two different polynucleotide sequences of interest;
2) causing the cleavage of said circular DNA molecules at a unique cleavage site into the vector molecule and at a unique cleavage site into the polynucleotide sequences of interest, thereby releasing linear cleavage fragments;
3) ligating said linear cleavage fragments to reassemble circular DNA molecules, thereby some of said resassembled circular DNA molecules have a recombined polynucleotide sequences of interest; and,
4) optionally, repeating steps (2) and (3) using other unique cleavage sites into the polynucleotide sequences of interest.

Optionally, the step (1) comprises introducing the at least two different polynucleotide sequences of interest into the vector molecule so as to obtain at least two circular DNA molecules, each comprising one of the at least two different polynucleotide sequences of interest.

In a most preferred embodiment, the cleavage of said circular DNA molecules is performed by endonuclease(s) specific of a unique endonuclease cleavage site into the vector molecule and endonuclease(s) specific of a unique endonuclease cleavage site into the polynucleotide sequences of interest.

In an alternative embodiment, the cleavage of said circular DNA molecules is performed by a step of PCR amplification of two fragments covering together said circular DNA molecules, and each fragment comprising a part of the polynucleotide sequence of interest and a part of the vector molecule. In a particular embodiment, each primer used in the step of PCR amplification comprises a unique endonuclease cleavage site, and the step of PCR amplification is followed by a step of cleavage by said endonuclease.

In an other alternative embodiment, the step (2) comprises the following steps : a) cleaving one of the at least two circular DNA molecules by endonuclease(s) at a first unique endonuclease cleavage site into the vector molecule and at a second unique endonuclease cleavage site into the polynucleotide sequences of interest, b) cleaving the other of the at least two circular DNA molecules by endonuclease(s) at the first unique endonuclease cleavage site into the vector molecule and at a third unique endonuclease cleavage site into the polynucleotide sequences of interest, c) treating the releasing linear cleavage fragments by a 3' exonuclease (3' ends trimming), and d) eliminating the 5' ovelapping single stranded ends.

Preferably, the method according to the present invention comprises an additional step of selecting recombined polynucleotide sequences of interest having an improved characteristic as compared to a reference polynucleotide sequence of interest.

The polynucleotide sequences of interest can be derived from genomic, metagenomic, environmental, plasmidic, mitochondrial or any complex DNA of plants, animals, insects, bacteria, cells, virus, phage or any existing organism. It can be generated by prior steps of DNA recombination, mutagenesis or synthesis. In a particular embodiment, the polynucleotide sequences of interest are metabolite(s) production pathway or part thereof. In an other particular embodiment, the polynucleotide sequences of interest encode a protein, preferably an enzyme. Optionally, the method of the present invention allows to shuffle protein domains, catalytic sites, affinity sites or any combination thereof.

Preferably, the vector molecule comprises at least one replication origin and at least one selection marker. In a particular embodiment, the vector molecule is cleaved into the at least one selection marker.

In a preferred embodiment, the endonucleases used to cleave the polynucleotide sequences of interest generate compatible ends for the ligation step (3), thereby allowing recombination to occur between polynucleotide sequences of interest. Preferably, the same endonuclease is used to cleave all the polynucleotide sequences of interest. Preferably, the endonucleases used to cleave the polynucleotide sequences of interest generate overhanging ends into the polynucleotide sequences of interest.

In a preferred embodiment, the endonuclease used to cleave the vector molecule generates blunt ends or overhanging ends which are not compatible with the ends into the polynucleotide sequences of interest for the ligation step (3).

Preferably, the ligating step is performed with a phage T4 DNA ligase or a thermostable ligase.

In a first embodiment, the step (2) of cleavage for each circular DNA molecule comprising one different polynucleotide sequence of interest is performed separately, and the step (3) of ligation is performed with a defined mixture of at least two products of individual cleavage of circular DNA molecules. In a second embodiment, the steps (2) and (3) are performed with a pool of circular DNA molecules.

Optionally, the method according to the present invention is performed by an automated device, the method comprising :
i) feeding the automated device with at least two circular DNA molecules, some cleavage reagents and some ligation reagents;
ii) running cleavage reaction(s);
iii) running ligation reaction(s); and
iv) optionally, repeating steps (ii) and (iii) with other cleavage reagents.

Optionally, the method according to the present invention is performed by a software automated device, the method comprising a previous step of determining by a software a sorted list of cleavage reagents.

### BRIEF DESCRIPTION OF THE DRAWINGS

The following drawings are illustrative of embodiments of the invention and are not meant to limit the scope of the invention as encompassed by the claims. Reference is made to the appended drawings :
**Figure 1 :** Random recombination of two polynucleotide sequences of interest.
**Figure 2 :** One example of the structure of a vector molecule.
**Figure 3 :** One example of the structure of a circular DNA molecule (association of a polynucleotide sequence of interest with a vector molecule).
**Figure 4** : Comparison of two random recombination of two polynucleotide sequences of interest with either successive cleavage or "split and mix" approach.
**Figure 5** : Random recombination of two polynucleotide sequences of interest with overlapping ends.
**Figure 6 :** Restriction map of pBR-Mut1 and pBR-Mut2.
**Figure 7** : Random recombination of two genes encoding for enzymes (beta-lactamases).
**Figure 8** : Shuffling of a polyketide synthase metabolic pathway.
**Figure 9 :** Sequence of *S. glaucescens* tetracenomycin C PKS gene clustery.
**Figure 10** : Map of *S*. *glaucescens* tetracenomycin C PKS gene cluster with unique restriction sites.

### DETAILED DESCRIPTION OF THE INVENTION

The method of the present invention relates to the recombination (or shuffling) of either high or low homology polynucleotide sequences of interest through steps of circular DNA molecules cleavage and ligation, thus generating recombined polynucleotide sequences of interest, some of which potentially coding for proteins with improved properties as compared to reference proteins.

On the opposite to usual DNA cloning that aims at creating a pre-defined DNA molecule, the method of the present invention randomly generates circular DNA molecules showing recombination events between different polynucleotide sequences of interest (see Figure 1).

The present invention concerns a method for *in vitro* recombining polynucleotides sequences of interest from at least two initial different polynucleotide sequences of interest by cleavage/ligation of circular DNA molecules, comprising :
1) providing at least two circular DNA molecules, each comprising a vector molecule and one of the at least two different polynucleotide sequences of interest;
2) causing the cleavage of said circular DNA molecules at a unique cleavage site into the vector molecule and at a unique cleavage site into the polynucleotide sequences of interest, thereby releasing linear cleavage fragments;
3) ligating said linear cleavage fragments to reassemble circular DNA molecules, thereby some of said resassembled circular DNA molecules have a recombined polynucleotide sequences of interest; and,
4) optionally, repeating steps (2) and (3) using other unique cleavage sites into the polynucleotide sequences of interest.

Optionally, the step (1) comprises introducing the at least two different polynucleotide sequences of interest into the vector molecule so as to obtain at least two circular DNA molecules, each comprising one of the at least two different polynucleotide sequences of interest. In a first embodiment, the circular DNA molecules comprise different vector molecules. In a preferred embodiment, the circular DNA molecules comprise the same vector molecule.

The method according to the present invention can involve at least 2, 3, 4, 5, 10, 20, 30, 50, 100, 200, 500 or 1000 different polynucleotide sequences of interest.

In a preferred embodiment, the present invention concerns a method *for in vitro* randomly recombining polynucleotides sequences of interest by circular DNA molecules cleavage/ligation, comprising:
1) introducing at least two different polynucleotide sequences of interest into a vector molecule so as to obtain at least two circular DNA molecules comprising one of the polynucleotide sequences of interest, wherein the vector molecule comprises at least one unique endonuclease cleavage site and the polynucleotide sequences of interest comprises at least one unique endonuclease cleavage site;
2) causing the cleavage of said circular DNA molecules at a unique endonuclease cleavage site into the vector molecule and at a unique endonuclease cleavage site into the polynucleotide sequences of interest, thereby releasing linear cleavage fragments;
3) ligating said linear cleavage fragments to reassemble circular DNA molecules, thereby some of said resassembled circular DNA molecules have a recombined polynucleotide sequences of interest; and,
4) optionally, repeating steps (2) and (3) using other unique endonuclease cleavage sites of the polynucleotide sequences of interest.

Optionally, products resulting from each step or cycle can be divided in separate tube and undergo different cleavage and/or ligation reactions to access several random recombination possibilities in parallel.

### Definitions

"Polynucleotide and polynucleotide sequence of interest" as used herein, refer to any nucleic or ribonucleic acid sequence, including mRNA and cDNA. A polynucleotide of interest may be or include a gene or a portion of a gene, an operon, a metabolic pathway or parts thereof. A polynucleotide or polynucleotide sequence of interest can be obtained in different ways, including extraction or amplification from a nucleic acid source, chemical synthesis and synthesis by polymerization.

"Gene" as used herein, refers to a polynucleotide or portion thereof associated with a known or unknown biological function or property. Thus genes include coding sequences, ORF (Open Reading Frames), regulatory sequences and recognition sequences. Under appropriate *in vivo* or *in vitro* conditions, some genes can encode a polypeptide.

"Metabolic pathway" as used herein, refers to series of genes that enable directly or indirectly chemical reactions, to achieve in either the formation of a metabolic product, or the initiation of another metabolic pathway (then called a flux generating step). Many of these pathways are elaborate, and involve a step by step modification of the initial substance to shape it into the product with the exact chemical structure desired.

"Operon" as used herein, refers to a group of nucleic acid sequences including an operator, a common promoter, and one or more structural genes that are controlled as a unit to produce messenger RNA (mRNA).

"Property / Improved property" as used herein, refer to the function that can be encoded by a polynucleotide sequence of interest. This property can for example be an enzymatic activity or an affinity if said sequences codes for a protein, or an endonuclease activity for example if said sequences codes for a catalytic mRNA, or a physico-chemical characteristic (size, folding, solubility, association and/or dissociation rate, chemical composition (C,N,H,O), isoelectric point, stability, pH sensitivity or optimum), or a production level (as for example the level of production of one protein if said sequence codes for this protein), or a therapeutic property if the polynucleotide sequence of interest is a metabolic pathway enabling production of active metabolites. An improved property as used herein refers to a property that differs from the corresponding property of at least one reference polynucleotide sequence of interest defined by the experimenter (for example higher or lower affinity or enzymatic activity of a protein, higher or lower expression yield of this protein, higher or lower size of the polynucleotide sequence, metabolites spectrum activity on anti-infective or anti-cancer targets ...).

*"In vitro",* as used herein, refers to any location outside a living organism, as opposite to *"in vivo".*

"Recombination / shuffling" as used herein, refers to the covalent association of a fragment of one polynucleotide sequence of interest with a fragment of another polynucleotide sequence of interest.

"Homologous polynucleotides" as used herein, refers to polynucleotide sequences which differ from each other at least at one corresponding residue position. Thus, as used herein, homologous encompasses what is sometimes referred to as partially heterologous. The homology, e.g., among the initial polynucleotide sequences, may range from 20 to 99.99% of identity, preferably 30 to 90% of identity, more preferably 40 to 80% of indentity. In some embodiments, the term homologous may describe sequences that are, for example, only about 20-45% identical at corresponding residue positions. Homologous sequences may or may not share with each other a common ancestry or evolutionary origin. "Identity" refers to sequence identity between two nucleic acid molecules. Identity can be determined by comparing a position in each sequence which may be aligned for purposes of comparison. When a position in the compared sequence is occupied by the same base, then the molecules are identical at that position. A degree of similarity or identity between nucleic acid sequences is a function of the number of identical or matching nucleotides at positions shared by the nucleic acid sequences. Various alignment algorithms and/or programs may be used to calculate the identity between two sequences, including FASTA, or BLAST which are available as a part of the GCG sequence analysis package (University of Wisconsin, Madison, Wis.), and can be used with, e.g., default settings.

"Heteroduplex polynucleotides" as used herein, refers to double-stranded polynucleotides in which the two strands are not perfectly complementary to each other.

"Vector molecule" as used herein, refers to one or several DNA molecule(s) that can be associated (for example, with a ligase) to a polynucleotide sequence of interest to obtain a circular DNA molecule. A vector molecule can be obtained in different ways, including extraction, amplification from a nucleic acid source, chemical synthesis and synthesis by polymerization. In a preferred embodiment, the vector molecule is a plasmid.

"Endonuclease" as used herein refers to an enzyme which makes a double-stranded break into a DNA molecule at highly specific locations. This endonuclease can be a "Restriction enzyme" or a "homing endonuclease". This endonuclease recognizes a specific target nucleotide sequence in DNA and break the DNA chain (double-strand break) at or near the target. Restriction enzymes and Homing Endonuclease are described by New England Biolabs (http://www.neb.com/nebecomm/products/categoryl.asp?).

By "unique endonuclease cleavage site" is intended herein that the vector molecule and/or the polynucleotide sequence of interest bears only one endonuclease cleavage site for a particular endonuclease. In a preferred embodiment, the unique endonuclease cleavage site for a particular endonuclease occurs only once in the circular DNA molecule comprising the vector molecule and the polynucleotide sequence of interest.

By "endonuclease cleavage site" is intended herein a sequence comprising the recognition site and the cleavage site of the endonuclease, especially if the two sites are distinct.

"Ligase" as used herein, refers to an enzyme or a chemical process that can link together DNA strands that have double-strand breaks (a break in both complementary strands of DNA) with either overhanging ends or blunt ends.

### Initial polynucleotide sequences of interest

The initial polynucleotide sequences of interest may be derived from plants, animals, insects, bacteria, cells, virus, phage or any existing organisms. It may also be artificial sequences obtained by chemical or enzymatic DNA synthesis. When polynucleotide sequences are RNA, they are converted into the corresponding DNA using for example a reverse transcriptase, or through chemical synthesis.

Polynucleotide sequences of interest may be derived from genomic, plasmidic, mitochondrial or any complex DNA and may include introns.

The polynucleotide sequences of interest can encode proteins, enzymes, metabolites pathways or parts thereof (including but not limited to anti-infectious, anti-cancer, anti-inflammation, anti-proliferative, cytotoxic, immunologic or any active compounds), metagenomic sequences or any combination thereof. Therefore, the invention is particularly useful to provide recombined polynucleotide sequences of interest from these origins. When polynucleotide sequences of interest encodes proteins, in particular enzymes, the method according to the present invention allows to shuffle protein domains, catalytic sites, affinity sites, or any combination thereof.

In a preferred embodiment, polynucleotide sequences of interest are metabolite(s) production pathway or part thereof. Accordingly, the method of the present invention comprises :
1) providing at least two circular DNA molecules, each comprising a vector molecule and one of the at least two different metabolite(s) production pathway sequences or part thereof;
2) causing the cleavage of said circular DNA molecules at a unique cleavage site into the vector molecule and at a unique cleavage site into the metabolite(s) production pathway sequences or part thereof, thereby releasing linear cleavage fragments;
3) ligating said linear cleavage fragments to reassemble circular DNA molecules, thereby some of said resassembled circular DNA molecules have a recombined metabolite(s) production pathway sequences or part thereof; and,
4) optionally, repeating steps (2) and (3) using other unique cleavage sites into the metabolite(s) production pathway sequences or part thereof.
Preferably, the metabolite(s) production pathway sequences are derived from metagenomic DNA. In a particular embodiment, metabolites resulting from recombined metabolites pathway sequences have an improved property as compared to reference metabolites.

The initial polynucleotide sequences of interest can be obtained through usual techniques such as restriction gene cloning, hybridization, amplification (PCR, RT-PCR, NASBA, Rolling circle, isothermal amplifications).

Polynucleotide sequences of interest may also be generated by prior rounds of shuffling, by the method of the present invention or by any other shuffling method (DNA-Shuffling, Step, L-Shuffling, ....).

These polynucleotide sequences of interest may also be derived from a parental polynucleotide sequence after a step of site directed mutagenesis or random mutagenesis or synthesis. In particular, endonuclease cleavage site(s) can be introduced into polynucleotide sequences of interest by using usual site directed mutagenesis methods, while preferably keeping the open reading frames and coding sequence of genes that are present in the polynucleotide sequences of interest.

Similarly, it may also comprise polynucleotide sequences of interest obtained after a selection step of polynucleotide sequences of interest having an improved property as compared to a reference polynucleotide sequence of interest .

In a preferred embodiment of the present invention, the polynucleotide sequences of interest can be one or several polynucleotide sequences derived from Metagenomics libraries created as described in patent U.S. 6,989,249. Such libraries contain high molecular weight DNA from environmental samples, to take advantage of genes and genetic pathways from yet untapped bacterial diversity. These high molecular weight DNA can be directly used as polynucleotide sequences, or preliminary cleaved in smaller pieces that are individually used as initial polynucleotide sequences of interest.

In a more specific embodiment of the technology, the polynucleotide sequences of interest are a specific portion of these high molecular weight DNA from environmental samples, such as a particular part of biosynthesis pathways of active molecules. These particular portions are used as initial polynucleotide sequences of interest in the method of the present invention, and the recombined progeny sequences are reintroduced in the entire biosynthesis pathway to enable the production of novel molecules from these modified pathways.

The polynucleotide sequences of interest can also be obtained through hybridization methods on membranes or on DNA-chips. In such an embodiment, clones (or DNA from said clones) from a library are gridded on membranes or on DNA-chips, and are screened as described in Maniatis *et al.* (Maniatis, , Sambrook, Fritsch, (1989). Molecular Cloning: A Laboratory Manual 2nd ed.), with specific probes to identify which clones share the same homologous sequence(s). DNA from said detected clones can then be recovered, optionally cleaved in smaller pieces and used as initial polynucleotide sequences of interest in the method of the present invention.

### Vector molecule

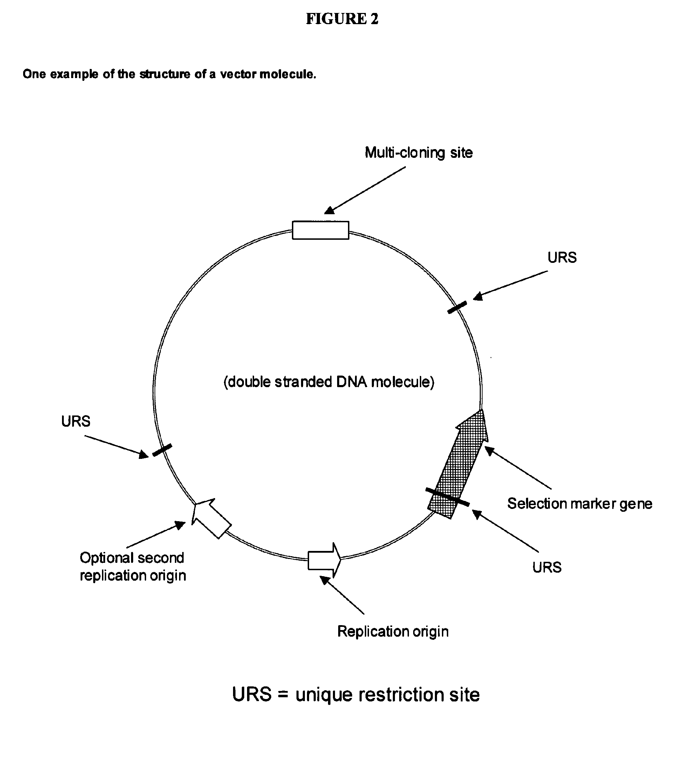

Said vector molecule is a linear or circular DNA molecule having :
- at least one replication origin; and
- at least one selection marker (including but not limited to antibiotic resistance genes, suppressor tRNA genes, reporter enzyme gene, auxotrophy complementary gene) that enables to detect cells that have acquired this vector molecule after a transfection, infection or transformation step (see Figure 2).

In a preferred embodiment, the vector molecule is a plasmid. It can be well known plasmids dedicated to different types of cells (bacteria, yeasts, fungi, mammalian cells, insect cells) such as pUC18 or pUC19, pBSKS^{™} (Stratagene), pET vector family (Novagen), pQE vector family (Qiagen), pBR322, pACYC184, pACYC177, pBeloBac11, pFOS1, pPIC vector family (InvitroGen), pFastBac^{™} vector family (InvitroGen), pCMV, pCR®-Blunt, pZERO® or pcDNA^{™} vector family (InvitroGen), or any derived vectors thereof.

The vector molecule can also be arms of phages, such as the lambda phage. In that case, each arm is ligated at the ends of the polynucleotide sequence of interest. After packaging of the linear DNA and infection, phage circularizes and replicates its DNA, thus providing a circular DNA molecule. Such vectors have been widely described (see for example the Zap Express^{™} vector system from Statagene).

In a specific embodiment, the vector molecule bears at least one unique endonuclease cleavage site. In a preferred embodiment, the vector molecule bears several unique endonuclease cleavage sites. In a most preferred embodiment, the unique endonuclease cleavage site is a rare restriction site. Accordingly, the preferred endonuclease is an endonuclease having a recognition sequence of at least 4, 6, 7, 8, 9 or 10 bases. For example, the endonuclease can be a restriction enzyme, such as BbvC I, Asc I, AsiS I, Fse I, Not I, Pac I, Pme I, Sbf I, SgrA I, Swa I, Sap I, CciN I, FspA I, Mss I, Sda I, Sgf I, Smi I, Srf I, and Sse8387 I. Alternatively, the endonuclease can be a homing endonuclease (see Flick et al., 1998, Nature, 394, 96). Examples of homing endonucleases are I-Ceu I, I-Cre I, I-Chu I, I-Csm I, I-Dmo I, I-Pan I, I-Sce I, I-Sce II, I-Sce III, I-Sce IV, F-Sce I, F-Sce II, PI-Aae I, PI-Ape I, PI-Ceu I, PI-Cir I, PI-Ctr I, PI-Dra I, PI-Mav I, PI-Mfl I, PI-Mgo I, PI-Mja I, PI-Mka I, PI-Mle I, PI-Mtu I, PI-MtuH I, PI-Pab III, PI-Pfu I, PI-Pho I, PI-Pko I, PI-Psp I, PI-Rma I, PI-Sce I, PI-Ssp I, PI-Tfu I, PI-Tfu II, PI-Tli I, PI-Tli II, PI-Tsp I, PI-Tsp II, PI-Bsp I, PI-Mch I, PI-Mfa I, PI-Mga I, PI-Mga II, PI-Min I, PI-Mma I, PI-Msh I, PI-Msm II, PI-Mth I, PI-Tag I, PI-Thy II, I-Ncr I, I-Ncr II, 1-Pan II, I-Tev I, I-Ppo I, I-Dir I, I-Hmu I, I-Hmu II, I-Tev II, I-Tev III, F-Sce I, F-Sce II (HO), F-Suv I, F-Tev I, and F-Tev II. Preferably, said homing endonuclease is chosen from the group consisting of I-Ceu I, II-Sce I, PI-Psp I, and PI-Sce I (available at New England Biolabs, cat. # R0699S, # R0694S, # R0695S and # R0696S, respectively).

This or these endonuclease cleavage sites may be preferably located into the selection gene. Accordingly, the selection gene can further allow to select the proper reassembled circular DNA molecules. Most preferably, the vector molecule comprises at least one unique endonuclease cleavage site which does not exist in the polynucleotide sequences of interest, so that man of the art can use endonucleases that selectively cleave either the vector molecule or the polynucleotide sequences of interest.

This or these endonuclease cleavage sites may be initially existing in the vector molecule or inserted using either site directed mutagenesis, any other well known methodology of mutagenesis (Splicing overlap PCR (SOE-PCR), *in vivo or in vitro* recombination), or by chemical or enzymatic DNA synthesis and/or assembly.

### Introduction of polynucleotide sequence of interest into the vector molecule

The initial polynucleotide sequences of interest can be inserted into a vector molecule by conventional molecular biology methods such as DNA cloning (see for a reference Maniatis, Sambrook, Fritsch, (1989). Molecular Cloning: A Laboratory Manual 2nd ed), *in vivo* or *in vitro* recombination (see for an example the Gateway^{™} system - InvitroGen - that relies on *in vitro* specific recombination events between *att*L and *att*R sites respectively located at the ends of the initial polynucleotide sequences and at the ends of the vector molecule), or transposition. Other association techniques can also be used to insert the initial polynucleotide sequences of interest into a vector molecule such as the TOPO® cloning method (Invitrogen) that relies on the use of a vector molecule with 3'-T overhanging ends activated with topoisomerase I for the direct cloning of initial polynucleotide sequences obtained by Taq PCR amplification, the Zero Background^{™} method (Invitrogen) that involves vector molecules bearing a bacterial toxic gene (ccdB) which is disrupted when the initial polynucleotide sequence is inserted in the vector (thus avoiding bacterial death after transformation of vector molecules in which initial polynucleotide sequences have been properly inserted).

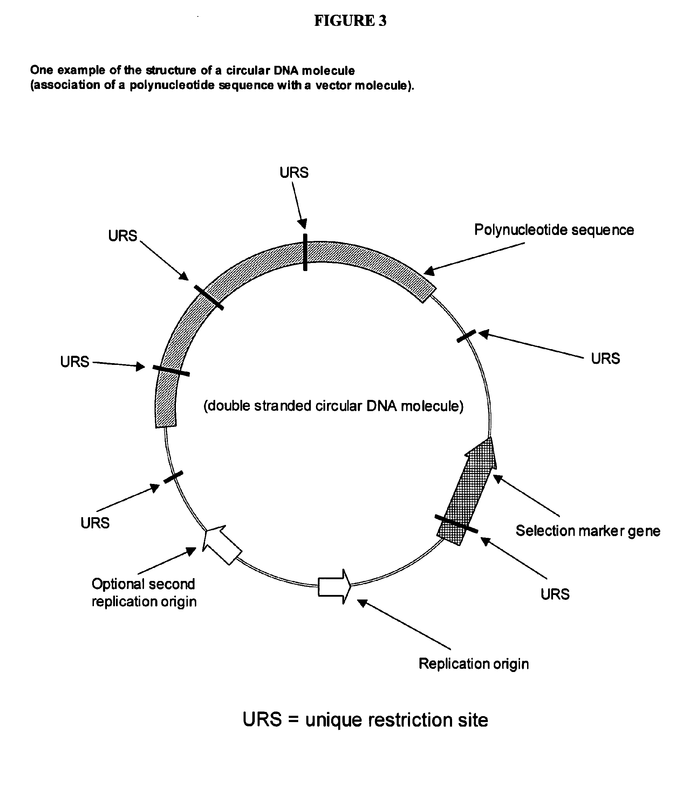

Said polynucleotide sequences of interest can also be introduced into the vector molecule through direct chemical or enzymatic DNA synthesis and/or assembly. This association generates a circular double stranded DNA molecule which contains the sequence of the vector molecule and the sequence of the polynucleotide sequence of interest (see Figure 3). The initial polynucleotide sequences of interest can be individually inserted into a vector molecule in separate reactions, or pooled together and inserted in a unique reaction. The different initial polynucleotide sequences can be inserted in the same type of vector molecule, or in different types, as long as the cleavage of the different vector molecules at step (2) generates compatible ends for the ligation at step (3) in order to reassemble circular DNA molecules.

### Cleavage

The cleavage may be achieved using enzymes (restriction enzymes, nucleases such as DNAse I), mechanic shearing of the DNA, UV treatment or chemicals treatment, PCR amplification, or any combination thereof.

However, in a preferred embodiment, the cleavage is achieved by endonucleases such as restriction enzymes or homing endonuclease (for a restriction database, see *Rebase* on http://rebase.neb.com/rebase/ or in the *Inbase* located on http://www.neb.com/neb/inteins.html). Some endonuclease, which can be used in the present invention, make strand incisions immediately opposite one another, producing "blunt end" DNA fragments. Some others, which can be preferably used in the present invention, make slightly staggered incisions, resulting in "sticky ends", out of which one strand protrudes. Because recognition sequences and cleavage sites differ between restriction enzymes, the length and the exact sequence of a sticky-end "overhang", as well as whether it is the 5' end or the 3' end strand that overhangs, depends on which enzyme produced it. Base-pairing between overhangs with complementary sequences enables two fragments to be joined or "spliced" by a DNA ligase at step (3). DNA-ligase can also splice blunt ends. A sticky-end fragment can be ligated not only to the fragment from which it was originally cleaved, but also to any other fragment with a compatible sticky end (for example ends generated by *Bcl* I are compatible with ends generated by *Bam* HI). If a restriction enzyme has a non-degenerate palindromic cleavage site, all ends that it produces are compatible. Obviously ends produced by endonucleases generating blunt ends are compatible with any other blunt ends. Ligation at step (3) of two compatible fragment ends can create a novel restriction site (for example ligation of *Bcl* I derived ends with *Bam* HI derived ends generates an *Alw* I restriction site), or eliminate one restriction site (for example ligation of *Bsp* HI derived ends with *Fat* I derived ends eliminates *Bsp* HI site while regenerating the *Fat* I site). So, in the present invention, knowledge of cleavage sites allows man of the art to anticipate which DNA fragments can be joined in which ways, and to choose appropriately endonucleases.

Endonucleases are selected to cleave at a unique site into the polynucleotide sequence of interest and at a unique site into the vector molecule, so as to obtain two fragments from the circular DNA molecule. The ends of the fragments generated by cleavage of one polynucleotide sequence of interest and its vector molecule must be compatible for the ligation at step (3) with the restriction ends of the fragments derived from the cleavage of at least another polynucleotide sequence of interest and its vector molecule.

In a most preferred embodiment, cleavage at step (2) is achieved in a way that favours ligation of vector molecule ends together on one hand and polynucleotide of interest ends together on the other hand at step (3). For this purpose, and when possible, the endonuclease cleavage site selected for the vector molecule differs from the one selected for the polynucleotide sequence of interest, so that an orientated ligation of DNA fragments can occur at step (3). Accordingly, in a preferred embodiment, the endonuclease selected for cleaving the vector molecule and the polynucleotide sequence of interest leads to uncompatible ends between the vector molecule and the polynucleotide sequence of interest.

The endonuclease cleavage site(s) into the polynucleotide sequences of interest are chosen to enable a recombination event of at least two polynucleotide sequences at the setp (3). Advantageously, the endonuclease cleavage site may be defined on the basis of an homology alignment of the polynucleotide sequences of interest : homologous regions share common endonuclease cleavage sites that can be used to cleave polynucleotide sequences of interest, and thus to recombine them after a ligation at step (3). Such a selection based on homology enables to keep the overall structure of gene domains during the recombination events. For low homology regions, endonuclease cleavage sites can also be used. They can, when necessary, be artificially incorporated using usual site directed mutagenesis methods. In this case, silent mutations are advantageously achieved to keep the open reading frame and coding sequence of genes that are present on the polynucleotide sequences of interest (for example the codons GCA(ala)-GGG(gly) are changed in GCC(ala)-GGC(gly) which is the recognition site of the *Nae* I restriction enzyme).

Selection and use of the different endonucleases enables to finely control the location of the desired recombination events, and the number of these recombination events. This control enables to recombine precisely polynucleotide of interest coding for proteins in order to shuffle protein domains, catalytic sites, affinity sites (such as variable regions of antibodies), and to rapidly create random chimeric proteins having the sequence of several different initial polynucleotide sequences of interest, or to create novel metabolic pathways having the sequence of several different initial biosynthesis pathways sequences.

In a preferred embodiment of the present invention, cleavage of the vector molecule is achieved with endonucleases generating blunt ends (e.g., Pme I, Swa I, FspA I, Mss I, Smi I, and Srf I), while cleavage of the polynucleotide sequences of interest is done with a endonucleases generating overhanging ends (e.g., BbvC I, Asc I, AsiS I, Fse I, Not I, Pac I, Sbf I, SgrA I, Sap I, CciN I, Sda I, Sgf I, and Sse8387 I). That way, ligation of vector ends can not occur with polynucleotide sequences ends.

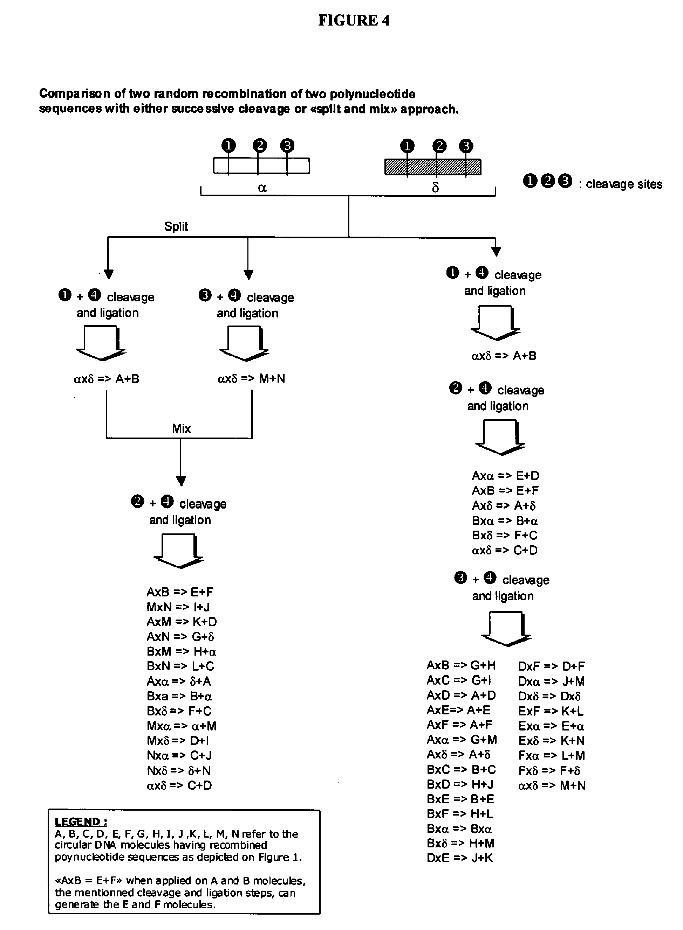

Cleavage of the circular DNA molecules can be run in a single reaction tube : the different circular DNA molecules are pooled together and undergo the same cleavage reaction(s). On the other hand, several parallel or successive cleavage reaction(s) can be run on each circular DNA molecule or on restricted pools of said circular DNA molecules. This embodiment enables to favour the random recombination of some circular DNA molecules that have experienced compatible cleavage reactions, introducing a desired bias during the random reassembly of these circular DNA molecules (see Figure 4). This is particularly useful to achieve the recombination of different families of initial polynucleotide sequences of interest at step (3) : some families are cleaved with a set of endonucleases, some others with another set of endonucleases, and all the corresponding linear fragments are pooled before step (3) for the ligation. Separate cleavage reactions of the circular DNA molecules can be used to favour the rapid creation and test of some particular recombined polynucleotide sequences of interest as compared to the whole theoretical population of recombined polynucleotide sequences of interest that can be generated through the successive cleavage/ligation steps (see Figure 4).

In one embodiment of the present invention, the cleavage in the step (2) of said circular DNA molecules is performed by a step of PCR amplification of two fragments covering together said circular DNA molecules, and each fragment comprising a part of the polynucleotide sequence of interest and a part of the vector molecule. In other words, the two fragments taking together comprise all the circular DNA molecules, and the first fragment contains a part of the polynucleotide sequence of interest and a part of the vector molecule and the second fragment contains the other part of the polynucleotide sequence of interest and the other part of the vector molecule. Optionally, the two fragments have overlapping parts into the polynucleotide sequence of interest and into the vector molecule.

Therefore, the present invention concerns a method *for in vitro* recombining polynucleotides sequences of interest from at least two initial different polynucleotide sequences of interest by cleavage/ligation of circular DNA molecules, comprising :
1) providing at least two circular DNA molecules, each comprising a vector molecule and one of the at least two different polynucleotide sequences of interest;
2) amplifying by PCR with two pairs of primers two linear fragments, covering together the circular DNA molecules and each fragment comprising a part of the polynucleotide sequence of interest and a part of the vector molecule;
3) ligating said linear fragments to reassemble circular DNA molecules, thereby some of said resassembled circular DNA molecules have a recombined polynucleotide sequences of interest; and,
4) optionally, repeating steps (2) and (3) using two other primers into the polynucleotide sequences of interest.

In one embodiment, the PCR amplification step is performed with two pairs of primers. In each pair of primers, a primer hybridizes in the polynucleotide sequence of interest and the other in the vector molecule. Each primer comprises a unique endonuclease cleavage site, and the step of PCR amplification is followed by a step of cleavage by said endonuclease. In another embodiment, PCR primers are degenerated and show a combination of endonuclease cleavage sites at their ends, so that amplified products from the same polynucleotide fragment will show different endonuclease cleavage sites at their ends.

These sites are cleaved with the endonucleases, and the corresponding fragments are used at step (3) to reassemble circular DNA molecules.

When necessary, said prepared linear fragments may be gel purified to avoid any contamination by the PCR template or by the small DNA pieces released by the restriction cleavage reaction.

PCR products may also be considered as cleavage products and used directly as blunt ends linear fragments at ligation at step(3).

In embodiments based on PCR cleavage at step (2), PCR amplified linear fragments may be separated in defined pools and used at step (3) to avoid the self reassembly of the initial circular DNA molecules. As an example, if the cleavage of each initial circular DNA molecules is achieved at step (2) by PCR amplification of two fragments, only one fragment amplified fragment from each circular DNA molecule is used at step (3), to exclude reassembly of the initial circular DNA molecules.

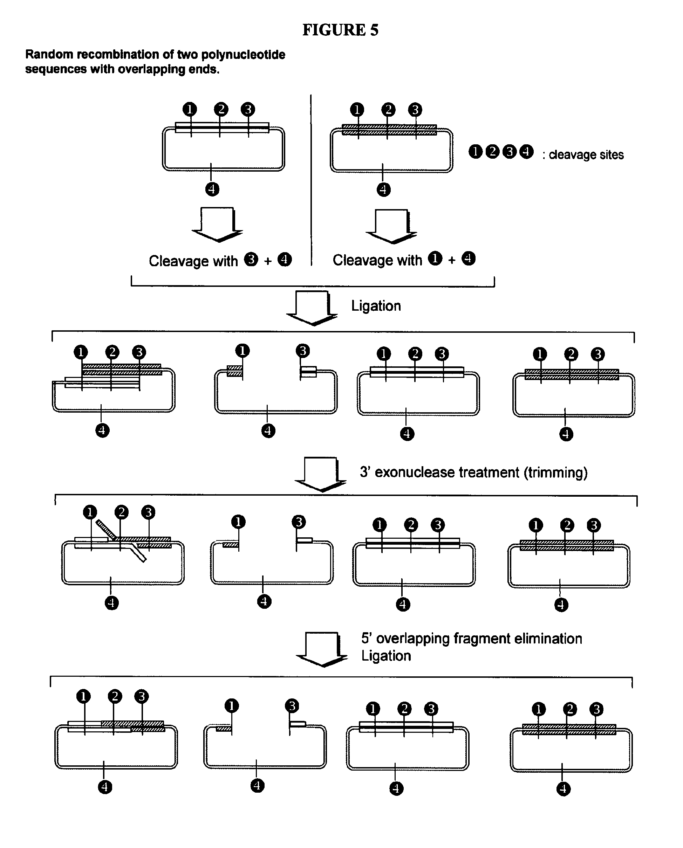

In a specific embodiment of the present invention, endonuclease cleavage sites used for the cleavage of the polynucleotide sequences of interest generates overlapping fragments at step (3) which are not directly compatible for a ligation. Then, a step of 3' ends trimming (with a 3' exonuclease such as *E*. *coli* exonuclease III) is run to provide long 5' single strand overlapping ends. This step is followed by a step of 5' overlapping single stranded ends cleavage to create sites compatible with ligation from overlapping linear fragment. The cleavage of 5' overlapping single stranded ends can be performed by a treatment with an endonuclease such as FEN-1 (also called RAD27/RTH1/MF-1/DNase IV, an endonuclease that cleaves the 5' overhanging structures generated by displacement synthesis when DNA polymerase encounters the 5' end of a downstream Okazaki's fragment) or *Mj* FEN-1 (Hwang et al., 1998, Nature Structural Biology, 5, 707 - 713) which releases the 5' overlapping single stranded ends and creates sites compatible with ligation (see Figure 5).

Therefore, the present invention concerns a method *for in vitro* recombining polynucleotides sequences of interest from at least two initial different polynucleotide sequences of interest by cleavage/ligation of circular DNA molecules, comprising :
1) providing at least two circular DNA molecules, each comprising a vector molecule and one of the at least two different polynucleotide sequences of interest;
2) causing the cleavage of said circular DNA molecules at a unique cleavage site into the vector molecule and at a unique cleavage site into the polynucleotide sequences of interest, thereby releasing linear cleavage fragments, wherein the step (2) comprises the following steps : a) cleaving one of the at least two circular DNA molecules by endonuclease(s) at a first unique endonuclease cleavage site into the vector molecule and at a second unique endonuclease cleavage site into the polynucleotide sequences of interest, b) cleaving the other of the at least two circular DNA molecules by endonuclease(s) at the first unique endonuclease cleavage site into the vector molecule and at a third unique endonuclease cleavage site into the polynucleotide sequences of interest, c) treating the releasing linear cleavage fragments by a 3' exonuclease (3' ends trimming), and d) eliminating the 5' ovelapping single stranded ends;
3) ligating said linear cleavage fragments to reassemble circular DNA molecules, thereby some of said resassembled circular DNA molecules have a recombined polynucleotide sequences of interest; and,
4) optionally, repeating steps (2) and (3) using other unique cleavage sites into the polynucleotide sequences of interest.

In a particular embodiment of this invention, cleavage is randomly achieved by a mechanic or enzymatic step (with DNAse I for example). In that case, cleavage of the circular DNA molecules generates overlapping fragments at step (3) that are not directly compatible for a ligation. Then a preliminary step of 3' ends trimming and a cleavage of 5' overlapping single stranded ends as described above is applied to recover ends that are compatible with a ligation at step (3).

Some restriction enzymes are sensitive to DNA methylation. By preparing the circular DNA molecules in strains that methylate or not, the experimenter can play on the same restriction sites, depending on their methylation state. For example, the GATC restriction site is not cleaved by the *Dpn* II enzyme when DNA is methylated, whereas it is cleaved by the *Sau* 3AI even if methylated. By selecting proper restriction sites and by preparing either methylated or non methylated DNA, some restriction sites will be usable, while others will not.

Even after an extensive cleavage reaction, some circular DNA molecules may remain uncleaved. To avoid any contamination of the random ligation by these uncleaved molecules, the linear fragments can be gel purified, and extracted using well known methods and kits (see Qiaquick^{™} kit - Qiagen - as an example). Purified linear fragments are then used for step (3).

At the end of the cleavage step, enzymes can be inactivated (heat or chemical treatment) to avoid any further cleavage activity during step (3).

### Ligation

Ligation at step (3) can be done with well known ligases such as phage T4 DNA ligase. Several ligation protocols or ligation kits (see Quick ligation^{™} kit from New England Biolabs) can be used for this step.

To avoid self reassembly of one circular DNA molecule during step (3), equimolar concentration of each linear DNA fragments is preferably used. The more different initial circular DNA molecules have been cleaved, the more different fragments are present in the ligation reaction, thus diluting linear fragments originating from the same circular DNA molecule and avoiding their self reassembly.

On the other hand, it may be interesting to introduce a statistical bias in the population of molecules through the incorporation of a higher amount of one or several specific linear fragments at step (3) or of one or several specific circular DNA molecules at step (2).

When necessary, fragments obtained after the cleavage reaction are either desalted, or gel purified or pooled before ligation. Desalting may be important if buffers used for the restriction enzymes are not compatible with the proper activity of the ligase.

In a preferred embodiment, cleavage reaction is run in buffers that are compatible with the ligase activity. As an example the following restriction buffers are compatible with the phage T4 ligase activity, when supplemented with 1mM ATP :
- Buffer 1 : 10mM Bis propane-HCl, 10mM MgCl₂, 1 mM DTT pH 7.0,
- Buffer 2 : 50 mM NaCl, 10 mM Tris-HCl, 10 mM MgCl₂, 1 mM DTT, pH 7.9,
- Buffer 3 : 100 mM NaCl, 50 mM Tris-HCl, 10 mM MgCl₂, 1 mM DTT, pH 7.9,
- Buffer 4 : 50 mM potassium acetate, 20 mM Tris-acetate, 10 mM Mg acetate, 1 mM DTT, pH 7.9.

Thermostable ligases can also be employed. In that case, ligation temperature is defined as a compromise between the annealing temperature of the restriction ends of the linear fragments and the optimal temperature of the enzyme. When such a compromise is not obvious, a temperature gradient can be achieved and repeated.

The use of thermostable ligases enables to achieve a preliminary heat inactivation of the cleavage enzymes in the presence of the ligase, thus making the whole process prone to automation.

The ligation products obtained at step (3) can be either pooled or split in different tubes before step (4).

In a particular embodiment, to avoid any contamination by linear molecules, circular DNA molecules obtained at sep (3) are purified.

Ligation of the linear DNA fragments can be followed in real time by sampling few microliters of the ligation reaction and analyzing them on an appropriate agarose gel. Linear fragments will migrate distinctly from circular ones, and should slowly disappear during the reaction to the benefit of circular DNA molecules apparition.

Ligation products can be separated the ones from the others by a serial dilution step, or by cell transformation. Cell transformation is achieved using either cell transfection for mammalian cells, or cell electroporation or cell chimio-transformation (see Maniatis, Sambrook, Fritsch, (1989). Molecular Cloning: A Laboratory Manual 2nd ed). Once cells have been transformed with the ligation product, each transformed cell has acquired a unique circular DNA molecule and can be isolated, by plating on agar plates for micro-organisms or by flux cytometry for mammalian cells for example. For micro-organisms, colonies can be manually or automatically picked (with a colony Picker such as the Qbot or the Qpix from Genetics) and cultured in a liquid medium to either produce the corresponding circular DNA molecules in larger quantities or to identify sequence(s) having an improved property as compared to reference sequence(s).

### Repeating

The recombination of polynucleotide sequences of interest can be further improved by repeating steps (2) and (3) of the present invention. During each cycle, new cleavage sites are selected to cut the recombined polynucleotide sequences of interest in fragments that will go through another ligation reaction to randomly reassemble them. Cycle after cycle of fragmentation and ligation, the level of random recombination is increased. New initial circular DNA molecule can be introduced at any cycle of this recombination process, depending on the availability of these molecules, or depending on the defined experimental strategy of the experimenter.

Therefore, the present invention concerns a method *for in vitro* recombining polynucleotides sequences of interest from at least two initial different polynucleotide sequences of interest by cleavage/ligation of circular DNA molecules, comprising :
1) providing at least two circular DNA molecules, each comprising a vector molecule and one of the at least two different polynucleotide sequences of interest, wherein the vector molecule comprises at least one unique endonuclease cleavage site and the polynucleotide sequences of interest comprises n unique endonuclease cleavage sites (X1, X2, ..., Xn);
2) causing the cleavage of said circular DNA molecules at a unique cleavage site into the vector molecule and at a unique cleavage site X1 into the polynucleotide sequences of interest, thereby releasing linear cleavage fragments;
3) ligating said linear cleavage fragments to reassemble circular DNA molecules, thereby some of said resassembled circular DNA molecules have a recombined polynucleotide sequences of interest; and,
4) repeating steps (2) and (3) using one of the unique cleavage sites X2, ..., Xn into the polynucleotide sequences of interest.

As in combinatorial chemistry, the products from each cycle can be divided in separate tube and undergo different cleavage reactions to access several random recombination possibilities in parallel. At each cycle, one or several reactions products can be pooled to gather these products in a new recombination round.

Before step (4), circular DNA molecules can be transformed or infected in cells to enable their amplification or the selection of the recombined polynucleotide sequences of interest as compared to a reference sequence.

Otherwise, additional circular DNA molecules can be introduced at any cycle of the recombination method according to the present invention.

### Selection of improved polynucleotide sequences

The method of the present invention is particularly useful to generate randomly recombined polynucleotide sequences of interest that show improved properties as compared to reference sequences. In nature, crossovers occur *in vivo* between genes, which lead to recombined genes cumulating properties of the two initial parental genes. This process is one of the main evolution mechanisms. As the present invention aims at randomly recombining *in vitro* polynucleotide sequences, it mimics the *in vivo* process, while accelerating it. Depending on the initial "parental" polynucleotide sequences of interest involved in the process, the randomly recombined sequences of interest obtained at step (3) and/or (4) can show improved properties as compared to reference sequences, optionally including the parental sequences.

The improved property can be a catalytic activity, a therapeutic activity, an affinity, a selectivity, a production yield, or a physico-chemical property. By "improved" is intended an increase or a decrease depending on the desired or undesired property to reach or to avoid.

The improved property can be :
(i) a modified property of the recombined sequence itself : physico-chemical property (size, pH, bases or amino acids composition, number of restriction sites, folding, solubility, structure, stability, association/dissociation constants), affinity property (avidity, specificity), or
(ii) a property of a compound synthesized or obtained directly or indirectly by the recombined sequence of interest such as : an activity (catalytic activity of the sequence(s) (if it(they) code for a ribozyme for example) or of the corresponding protein), an affinity of such compound (antibodies example), a selectivity of such compound (enzyme or antibody affinity), a production yield of such compound (production yield of a protein for example), a production yield of metabolite(s) synthesized thanks to the genetic information of the sequence (examples of secondary metabolites operons such as erythromycine), the nature of said compound (production of new metabolites from the recombined sequence for example), the activity of said compound (enzyme activity or therapeutic activity, spectrum of secondary metabolites on anti-infective or anti-cancer targets), a physico-chemical property (size, mass, isoelectric point, amino acid composition, C-N-H-O composition, structure, association/dissociation constants, hydrophobicity, immunogenicity, ...).

Then in a specific embodiment, the invention present invention concerns a method for *in vitro* randomly recombining polynucleotides sequences of interest by recursive circular DNA molecules cleavage/ligation, comprising :
1) introducing at least two different initial polynucleotide sequences of interest into a vector molecule so as to obtain at least two circular DNA molecules comprising one of the initial polynucleotide sequences of interest, wherein the vector molecule comprises at least one unique endonuclease cleavage site and the initial polynucleotide sequences of interest comprises at least one unique endonuclease cleavage site;
2) causing the cleavage of said circular DNA molecules at a unique endonuclease cleavage site into the vector molecule and at a unique endonuclease cleavage site into the initial polynucleotide sequences of interest, thereby releasing linear cleavage fragments;
3) ligating said linear cleavage fragments to reassemble circular DNA molecules, thereby some of said resassembled circular DNA molecules have a recombined polynucleotide sequences of interest;
4) repeating steps (2) and (3) using other unique endonuclease cleavage sites of the polynucleotide sequences of interest; and,
5) selecting recombined polynucleotide sequences of interest having improved characteristics as compared to reference polynucleotide sequences of interest.

In an alternative embodiment, the method comprises :
1) introducing at least two different initial polynucleotide sequences of interest into a vector molecule so as to obtain at least two circular DNA molecules comprising one of the initial polynucleotide sequences of interest, wherein the vector molecule comprises at least one unique endonuclease cleavage site and the initial polynucleotide sequences of interest comprises at least one unique endonuclease cleavage site;
2) causing the cleavage of said circular DNA molecules at a unique endonuclease cleavage site into the vector molecule and at a unique endonuclease cleavage site into the initial polynucleotide sequences of interest, thereby releasing linear cleavage fragments;
3) ligating said linear cleavage fragments to reassemble circular DNA molecules, thereby some of said resassembled circular DNA molecules have a recombined polynucleotide sequences of interest;
4) selecting recombined polynucleotide sequences of interest having improved characteristics as compared to reference polynucleotide sequences of interest; and,
5) repeating steps (2) to (4) using other unique endonuclease cleavage sites of the polynucleotide sequences of interest.

Recombined sequences having said improved property are identified through a selection step, using a reference sequence for comparison. Said selection can be achieved by nucleic acid hybridization, RFLP, DGGE, sequencing, electrophoresis, detection of homologous or recombinant expression (including detection of gene product), function (enzymatic activity), detection with antibodies (ELISA, western blots), enzymatic measurement, binding test, affinity test, stereoselectivity test, temperature or pH or salt or solvent sensitivity test, enzyme product(s) and or substrate(s) concentration(s) sensitivity test, aggregation test, solubility test, anti-infective test, anti-inflammatory test, anti-cancer test, anti-proliferative test, cytotoxicity test, immunology or immunogenicity test, cell based assays, physico-chemical measurements, or by any method known from the man of the art that enables to identify an improved property of a sequence or of its corresponding product(s) as compared to a reference sequence and/or of its product(s).

In a particular embodiment, the reference sequence can be the wild-type sequence. In an other particular embodiment, the reference sequence can be an initial polynucleotide sequence of interest used the method of the invention.

Therefore, these methods are methods for generating or preparing or selecting recombined polynucleotide sequences of interest having improved characteristics as compared to reference polynucleotide sequences of interest.

### Automation of said method

The method of the present invention can easily be automated, as it only involves liquid handling steps. Several commercially available liquid handling systems can be utilized to run the automation of this process (see liquid handlers from Perkin-Elmer, Caliper Life Sciences, Tecan, Eppendorf, Apricot Design, Velocity 11 as examples).

In one example of automation, a microtiter plate is prepared with each initial circular DNA molecule in a well, and placed on the liquid handler near another microtiter plate containing the endonucleases and the corresponding buffers stored at +4°C. The liquid handler launches the desired cleavage reaction in a third microtiter plate, which is either manually or automatically incubated at the appropriate temperature. If endonucleases with different reaction temperature are used, several "cleavage" microtiter plates can be created and incubated at the corresponding temperature. When necessary, cleavage reaction are stopped by heat treatment (using a specific heating bath on the liquid handler or a PCR machine placed on the table of the liquid handler), and optionally desalted (using well known DNA purification kits for automates - see the Qiagen purification kits for example). Linear DNA fragments are then mixed in the desired order in well of a new microtiter plate, and the liquid handler adds ligase and its reaction buffer to achieve ligation. After defined incubation conditions and time, the ligation products are optionally desalted and can undergo a novel round of cleavage / ligation as programmed by the experimenter on the liquid handler computer.

At the end of the recursive cleavage/ligation cycles, the recombined sequences of interest can be desalted and then automatically incubated with chimio-competent cell to obtain transformants.

The process of the invention can also be run on nano-devices. Such nano-devices can be micro-fluidic devices as described in patent FR2795426 or any other kind of "Lab-on-Chip" such as devices described in patent WO2005042163. These nano-devices will basically achieve the same automation sequence than a liquid handler, but at a smaller scale, thus saving space, reagents and optionally time (reaction at smaller volumes are usually more rapid).

Then, in a specific embodiment, the present invention concerns a method *for in vitro* randomly recombining polynucleotides sequences of interest by automated recursive circular DNA molecules cleavage/ligation, comprising :
1) feeding an automated device with at least two circular DNA molecules comprising polynucleotide sequences of interest, some cleavage reagents and some ligation reagents,
2) running cleavage reaction(s) with said cleavage reagents on said automated device of said circular DNA molecule at a unique endonuclease cleavage site into the vector molecule and at a unique endonuclease cleavage site into the initial polynucleotide sequences of interest, thereby releasing linear cleavage fragments;
3) running ligation reaction(s) on said automated device of said linear cleavage fragments to reassemble circular DNA molecules, thereby some of said resassembled circular DNA molecules have a recombined polynucleotide sequences of interest; and,
4) repeating steps (2) and (3) using other cleavage reagents of the polynucleotide sequences of interest.
   The circular DNA molecules comprising polynucleotide sequences of interest according to the present invention is a vector molecule comprises at least one unique endonuclease cleavage site and the initial polynucleotide sequences of interest comprises at least one unique endonuclease cleavage site.

The automation of the process enable to achieve in parallel a higher number of cleavage and ligation conditions while saving man power. All the more, automation increases reproducibility and quality of the recombination experiments.

### Software assisted method

The initial analysis of polynucleotide sequences of interest to select the proper endonucleases and their successive or parallel use for the cleavages can also by automatised thanks to a bio-informatic software. In that case, the sequences of the initial polynucleotides of interest are entered into the software, and the program identifies, using a restriction site database (as described in the *Rebase* on http://rebase.neb.com/rebase/ or in the *Inbase* located on http://www.neb.com/neb/inteins.html), restriction sites that can be used together or successively for an optimized recombination result. This restriction sites analysis can also take into account the alignment of the sequences to keep the overall structure of the sequences during recombination. The software generates a result list comprising endonucleases to be involved in the method of the present invention and the way they should be used. These results can be directly converted into a computer program sequence that will drive the motions of a liquid handler to execute the whole process.

Therefore, the present invention concerns a method *for in vitro* randomly recombining polynucleotides sequences of interest by automated recursive circular DNA molecules cleavage/ligation, comprising:
1) determining by a software a sorted list of cleavage reagents,
2) feeding an automated device with at least two circular DNA molecules comprising polynucleotide sequences of interest, said cleavage reagents and some ligation reagents,
3) running cleavage reaction(s) with said cleavage reagents on said automated device of said circular DNA molecule at a unique endonuclease cleavage site into the vector molecule and at a unique endonuclease cleavage site into the initial polynucleotide sequences of interest, thereby releasing linear cleavage fragments;
4) running ligation reaction(s) on said automated device of said linear cleavage fragments to reassemble circular DNA molecules, thereby some of said resassembled circular DNA molecules have a recombined polynucleotide sequences of interest; and,
5) repeating steps (3) and (4) using other cleavage reagents of the polynucleotide sequences of interest as determined in step 1).

### Advantages Of The Method Of The Present Invention

The advantages of the method of the present invention over pre-existing recombination methods appears clearly from the previous descriptions and embodiments. A non exhaustive list of others advantages of the present invention are described below :
1. As the method of the present invention does not involve any polymerase during the recombination step, no extra mutations are incorporated, which increases the number of active clones, when the process is applied to genes encoding for either proteins or synthesis pathways.
2. The process of the present invention is simple and easy to launch at the laboratory scale. It relies on recursive cleavage and ligation steps of circular DNA molecules to randomly recombine polynucleotide sequences. Man of the art selects the appropriate cleavage sites to ensure the adequate recombination.
3. By defining specific sets of endonucleases, one can control the location of the recombination events and the number of these recombination sites, so that protein or gene domains can either be shuffled or kept out of the recombination process.
4. As the recombination process of the present invention does not rely on a polymerisation step, nor on any hybridization step, it enables to shuffle very large polynucleotide sequence of interest, including operons, high molecular weight enzymes, multiple domains enzymes or enzymatic complexes, complete or partial metabolic synthesis pathways. In addition, there is no requirement of a minimal homology between the polynucleotides sequences of interest.
5. The whole process of the invention can be run at a unique temperature (cleavage and ligation at 30 or 37°C for example), which simplifies incubation steps and the set-up of the experiment.
6. The method of the present invention is prone to automation due to its simplicity, either on usual liquid handlers or on nano-devices. The automation can be fully integrated with a preliminary software assisted selection of the list of the cleavage enzymes, that will drive the liquid handler or the nano-device.
7. When cleavage enzymes are selected on the basis of sequence alignments of the poynucleotide sequences of interest, the method of the present invention enables to randomly recombine these polynucleotides while keeping the overall structure of these sequences (recombination occurs at homologous regions).
8. The steps of the process of the invention can be carried out successively without interruption by the same operator, advantageously on an automated device integrating each of the steps, or can be carried out in a discontinuous fashion, optionally by different operators.
9. Due to the creation and/or destruction of restriction cleavage sites during the random recombination of the polynucleotide sequences, a quality control of the reaction can be achieved at each cycle of fragmentation/ligation with very simple protocols such as RFLP. This enables to validate the proper recombination of the polynucleotide sequences before any further round of cleavage/ligation. Any other embodiments and advantages of the present invention will appear from the following examples, that are illustrative of embodiments of the invention and are not meant to limit the scope of the invention as encompassed by the claims.

### EXAMPLES

### EXAMPLE 1 : Generation of recombined genes through successive cleavage/ligation reactions

To ensure that recombination occurs properly with the process of the present invention, an experiment has been run with two parental genes, which recombination products show distinct phenotypes.

TEM-1 is an antibiotic resistance enzyme that cleaves beta-lactamines (*i.e.,* penicillins and cephalosporins) thus inactivating their anti-infective effect. Bacteria that have acquired this gene (called *bla*) are resistant to usual antibiotic treatments.

TEM-1 enzyme has been extensively studied and used as a simple model for random mutagenesis (Zaccolo and Gherardi, 1999, J Mol Biol., 285(2):775-83), codon scanning (Lefèvre et al., 1997, Nucleic Acids Res., 25(2):447-8) and directed evolution experiments (Stemmer, 1994, Nature, 370(6488):389-91), as TEM-1 mutants show different resistance profiles against beta-lactamines. For example, mutant G238S has been described as implied in cefotaxime hydrolysis and resistance (Saves et al., 1995, Biochemistry, 34(37):11660-7), while mutation N276D leads to clavulanic acid resistance (Saves et al., 1995, J Biol Chem., 270(31):18240-5).

### Construction of the initial circular DNA molecules :

Two mutants of the TEM-1 gene have been constructed :
- mutant 1 shows mutation S70F (according to Ambler's numbering of amino acids - Ambler R.P. et al., 1991, Biochem. J., 276, 269-272), which inactivates the catalytic serine implied in beta-lactamine deacylation (Sigal et al., 1982, Proc. Natl. Acad. USA, 79: 7157-60)). When transformed in bacteria, this deactivated mutant gene does not lead to any resistance.
- mutant 2 has two mutations : G238S, which enables cefotaxime resistance, and E166A which inactivates glutamic acid 166 implied in the catalytic activity (Adachi et al., 1991, J. Biol. Chem., 266:3186-91). As for mutant 1, this deactivated mutant gene does not lead to any resistance.

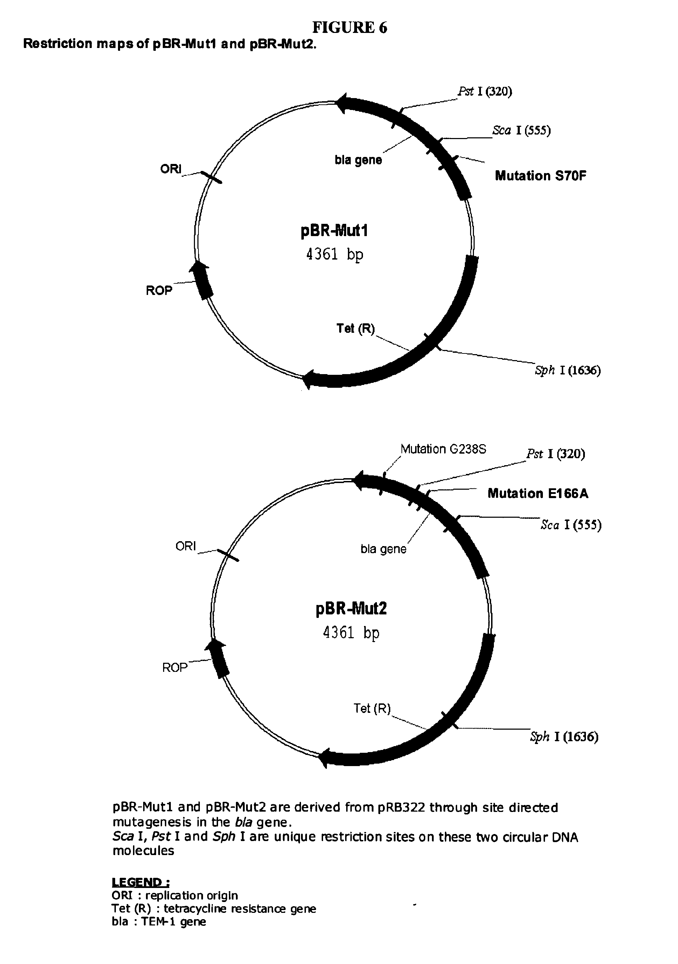

These two mutants have been built by site directed mutagenesis using the Quick Change Site-Directed mutagenesis kit from Strategene, starting from the pBR322 vector (amino acid mutations S70F and E166A were generated by nucleic acid mutations (A202T + C203T) and A491C respectively). The corresponding plasmids have been called pBR-Mut1 and pBR-Mut2 (see Figure 6).

### Cleavage of the circular DNA molecules in two conditions (first cycle) :

After a DNA preparation of these two plasmids with the Qiagen Midi Kit, 150 ng of each plasmid have been mixed in two separate tubes in order to have :
- one tube (tube 1) with a final concentration of 50 mM NaCl, 10 mM Tris-HCl, 10 mM MgCl₂, 1 mM DTT, pH 7.9, 10 units of *Sph* I (NEB ref #R0182S) and 10 units of *Sca* I (NEB ref #R0122S), in a volume of 25 µl.
- the other tube (tube 2) with a final concentration of 50 mM NaCl, 10 mM Tris-HCl, 10 mM MgCl₂, 1 mM DTT, pH 7.9, 10 units of *Sph* I and 10 units of *Pst* I (NEB ref #R0140S), in a volume of 25 µl.

After 60 minutes of incubation at 37°C, 2 µl of each mix were analyzed on a TBE agarose gel to check that a complete digestion of the plasmids had occurred.
Then tubes were incubated at 80°C for 30 minutes to heat inactivate restriction enzymes.

### Ligation of the linear fragments from each cleavage condition (first cycle) :

After cooling the tubes on ice, one microliter of T4 DNA ligase (NEB ref #M0202S) was added in each tube with a final ATP concentration of 1mM.
Tubes were then incubated at 16°C for one night. *E. coli* DH5α chimio competent cells were then transformed with the resulting ligation products, and plated on LB agar plates containing 12.5 µg/ml of tetracycline. Plates were incubated overnight at 37°C.

### Selection of the recombinant circular DNA molecules (first cycle) :

Colonies obtained on each plate were picked and replicated on a LB agar plate containing 12.5 µg/ml of tetracycline and 10µg/ml of ampicilline, and on a LB agar plate containing 12.5 µg/ml of tetracycline and 15 ng/ml of cefotaxime.

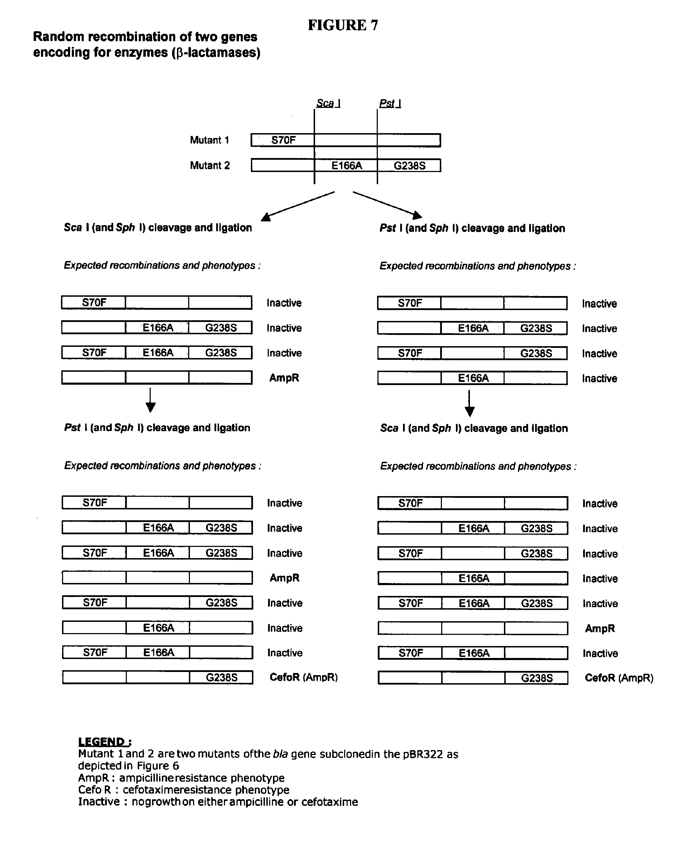

As expected by the random recombination reaction (see Figure 7), no growth was observed on cefotaxime plates for colonies originating from ligation products of tube 1 and 2, no growth was observed on ampicilline plates for colonies originating from ligation products of tube 2, whereas some colonies originating from ligation products of tube 1 grew on ampicilline plates.

To continue the random recombination process, a second cycle of cleavage and ligation has been achieved, by recovering the circular DNA molecules from the two pools of colonies plated on tetracycline only, and cleaving them with another restriction enzyme mix.

### Cleavage of the circular DNA molecules in two conditions (second cycle) :

Colonies obtained from ligation reaction of tube 1 were collected and incubated in LB medium (with 12,5 µg/ml of tetracycline) at 37°C, before extracting the corresponding plasmidic DNA mix with the Qiagen Midi Kit. 300 ng of this mix were incubated in a volume of 25 µl at a final concentration of 50 mM NaCl, 10 mM Tris-HCl, 10 mM MgCl₂, 1 mM DTT, pH 7.9, 10 units of *Sph* I and 10 units of *Pst* I.

The same work was achieved with colonies obtained from ligation reaction of tube 2, and 300 ng of this second plasmid mix were incubated in a volume of 25 µl at a final concentration of 50 mM NaCl, 10 mM Tris-HCl, 10 mM MgCl₂, 1 mM DTT, pH 7.9, 10 units of *Sph* I and 10 units of *Sca* I.

After 60 minutes of incubation at 37°C, 2 µl of each cleavage mix were analyzed on a TBE agarose gel to check that a complete digestion of the plasmids had occurred.
Then tubes were incubated at 80°C for 30 minutes to heat inactivate restriction enzymes.

### Ligation of the linear fragments from each cleavage condition (second cycle) :

After cooling the tubes on ice, one microliter of T4 DNA ligase was added in each tube with a final ATP concentration of 1mM.
Tubes were then incubated at 16°C for one night. *E. coli* DH5α chimio competent cells were then transformed with the resulting ligation products, and plated on LB agar plates containing 12.5 µg/ml of tetracycline. Plates were incubated overnight at 37°C.

### Selection of the recombinant circular DNA molecules (second cycle) :

Colonies obtained on each plate were picked and replicated on a LB agar plate containing 12.5 µg/ml of tetracycline and 10µg/ml of ampicilline, and on a LB agar plate containing 12.5 µg/ml of tetracycline and 15 ng/ml of cefotaxime.

As expected for this second random recombination reaction (see Figure 7), growth was observed on cefotaxime plates and on ampicilline plates for colonies originating from each ligation products.

This example demonstrates first that random recombination of genes can be achieved through recursive cleavage/ligation of circular DNA molecules, generating active and/or cefotaxime resistant mutants through the recombination of inactive genes. All the more, specific recombination products (such as cefotaxime resistant mutants) can be obtained selectively -if desired- through the appropriate selection and use of restriction enzymes (see results of cycle 1 in reaction tube#1). This would have not been possible when using the prior art shuffling technologies such as DNA shuffling, as all the genes are randomly cleaved and reassociated through hybridization and polymerization steps, thus generating a pool of polynucleotide sequences with no possible control over the recombination events.

### EXAMPLE 2 : Shuffling of a metabolic pathway (with intermediate amplification of recombined libraries)

Polyketides are an extensive class of structurally diverse natural products which provide a staggering range of clinically effective drugs. These include antibiotics (erythromycin A, rifamycin S), immunosuppressants (rapamycin, FK506), antifungals (amphotericin B), antiparasitics (avermectin), insecticidals (spinosyn A) and anticancers (doxorubicin, epothilones, discodermolide). They are a rich source of antibiotics, anti-cancer agents, cholesterol-lowering drugs, and other pharmaceuticals. The market for these drugs is more than $15 billion per year.

Polyketides are produced by the stepwise condensation of simple carboxylic acid precursors, in a fashion similar to fatty acid biosynthesis. The polyketide chains so formed are in many cases cyclised to produce macrocyclic rings. Their structural complexity is increased by the frequent presence of multiple chiral centres and by the addition of sugars, methyl and hydroxyl groups.

As polyketides are produced on "assembly lines" consisting of sets of proteins and enzymatic activities known as modules, which are directed by "gene clusters" in the host organism (Hopwood, 1997, Chem. Rev., 97(7):2465-2498), the number and nature of modules can be manipulated to engineer novel compounds, by randomly recombining the set of enzymes or modules from one or from several polyketide(s) "assembly line(s)".

If random mutation is a powerful tool to mutate genes and metabolic pathways, it has been extensively demonstrated that there is an optimal number of mutations that can be introduced in a gene to get the highest number of improved genes in the final library (see Chodorge et al., 2005, Adv. Synth. Catal., 347, 1022-1026). All the more, the improvement of genes through cumulative random mutations cycles rapidly reaches a maximum due to accumulation of deleterious mutations.

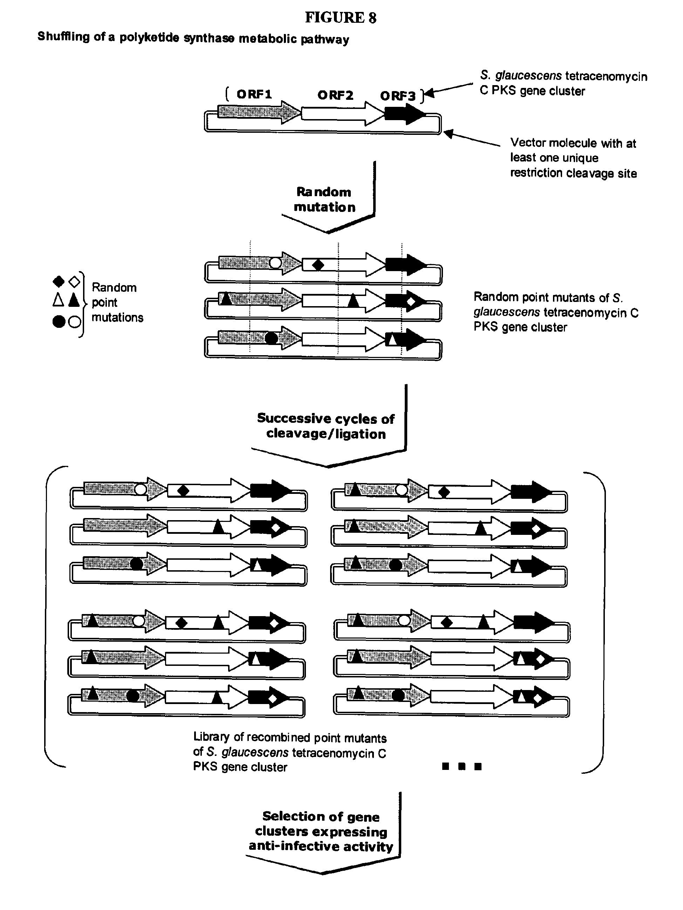

In this example, we take advantage of first randomly creating mutations on a metabolic pathway and then increasing this initial genetic diversity by the method of the present invention. For this purpose, one polyketide metabolic pathway for tetracenomycin C is first cloned, and the minimal PKS sequence is randomly mutated. These mutants are recombined to shuffle the initial mutations and create a higher genetic multiplicity. Then these shuffled minimal PKS are reintroduced in the initial complete pathway to be expressed in *Streptomyces,* and tested for the production of anti-infective compounds against *B. subtilis* (see figure 8).

### Construction of the initial circular DNA molecule :

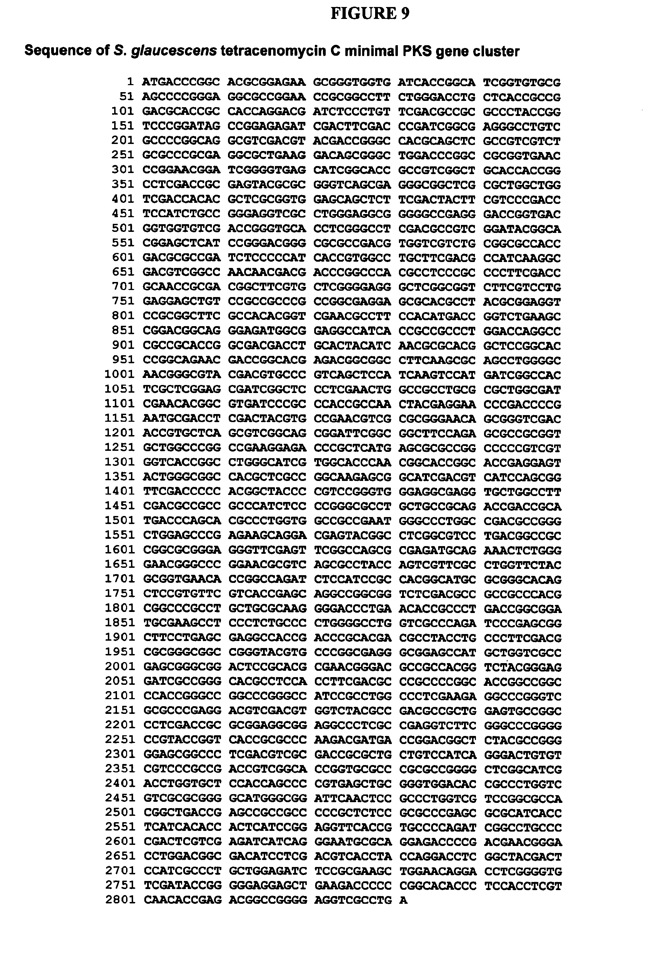

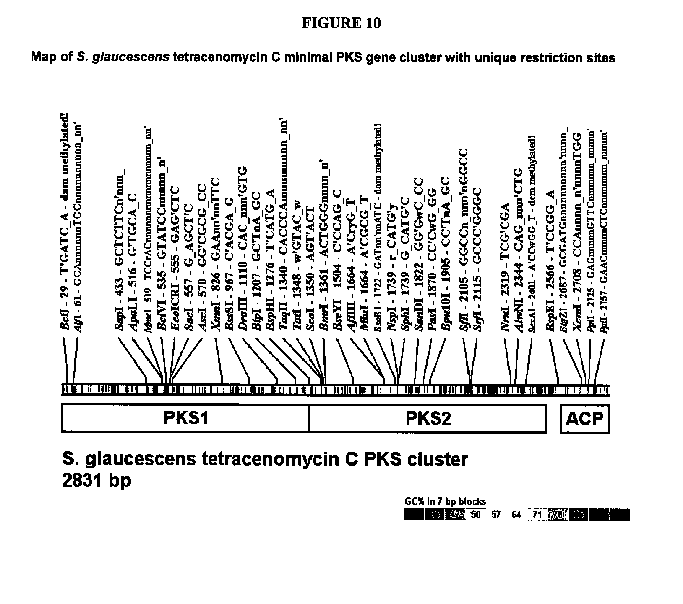

The sequence shown in figure 9 of S. *glaucescens* tetracenomycin C minimal PKS gene cluster is cloned as a blunt PCR product of 2831 bases in a pUC19 vector, preliminary cleaved with the *Pvu* II restriction enzyme. The corresponding circular DNA molecule shows at least one single restriction site on the vector part (such as *Xmn* I restriction site) and several single restriction sites on the PKS cluster (see figure 10).

This circular vector molecule is transformed in *E. coli* XL1-Red strain (a *mut*S, *mut*D, *mut*T defective strain from Stratagene, making its mutation rate approximately 5,000-fold higher than that of its wild-type parent) to generate *in vivo* random mutations on the circular DNA molecule, following the manufacturer's instructions. The resulting mutated circular DNA molecules are then recovered with an alkaline lysis extraction method for plasmids.

### First cleavage of the circular DNA molecules (shuffling of the first ORF of the cluster) :

After a DNA preparation of these mutated circular DNA molecules with the Qiagen Midi Kit, 200 ng of the mutated plasmid pool are mixed in one tube in a final volume of 100 µl and treated with *Xmn* I (NEB ref#R0194S) and *Apa* LI (NEB ref#R0507S) restriction enzymes in a final mix of 50 mM potassium acetate, 20 mM Tris-acetate, 10 mM Mg acetate, 1 mM DTT, pH 7.9, 100µg/ml BSA.
After 60 minutes of incubation at 37°C, 2 µl of the mix are analyzed on a TBE agarose gel to check that a complete digestion of the plasmids has occurred.
Then tube is incubated at 80°C for 30 minutes to heat inactivate restriction enzymes.

### Ligation of the linear fragments from the first cleavage condition :

After cooling the tubes on ice, one microliter of T4 DNA ligase (NEB ref #M0202S) is added in each tube with a final ATP concentration of 1mM.
Tube is then incubated at 16°C for one night. *E. coli* DH5α chimio competent cells are then transformed with the resulting ligation products, and plated on LB agar plates containing 10 µg/ml of ampicilline. Plates are incubated overnight at 37°C.

### Second cleavage of the circular DNA molecules (shuffling in the second ORF of the cluster) :

To continue the random recombination process, a second cycle of cleavage and ligation is achieved, by collecting all the circular DNA molecules from the first ligation reaction, and cleaving them with another restriction enzymes mix.
For this purpose, *E*. *coli* DH5α transformed with the ligation product from first cleavage reaction, are recovered and pooled, and the corresponding recombined circular DNA molecules are extracted with the Qiagen Midi Kit.
200 ng of the recombined plasmid pool are mixed in one tube in a final volume of 100 µl and treated with *Xmn* I (NEB ref#R0194S) and *Sph* I (NEB ref#R0182S) restriction enzymes in a final mix of 50 mM potassium acetate, 20 mM Tris-acetate, 10 mM Mg acetate, 1 mM DTT, pH 7.9, 100µg/ml BSA.
After 60 minutes of incubation at 37°C, 2 µl of the mix are analyzed on a TBE agarose gel to check that a complete digestion of the plasmids has occurred.

Then tube is incubated at 80°C for 30 minutes to heat inactivate restriction enzymes.

### Ligation of the linear fragments from the second cleavage condition :

After cooling the tubes on ice, one microliter of T4 DNA ligase (NEB ref #M0202S) is added in each tube with a final ATP concentration of 1mM.
Tube is then incubated at 16°C for one night. *E. coli* DH5α chimio competent cells are then transformed with the resulting ligation products, and plated on LB agar plates containing 10 µg/ml of ampicilline. Plates are incubated overnight at 37°C.

### Third cleavage of the circular DNA molecules (shuffling in the third ORF of the cluster):

To finalize the random recombination process, a third cycle of cleavage and ligation is achieved, by collecting all the circular DNA molecules from the second ligation reaction, and cleaving them with another restriction enzymes mix.
For this purpose, *E. coli* DH5α transformed with the ligation product from second cleavage reaction, are recovered and pooled, and the corresponding recombined circular DNA molecules are extracted with the Qiagen Midi Kit.
200 ng of the recombined plasmid pool are mixed in one tube in a final volume of 100 µl and treated with *Xmn* I (NEB ref#R0194S) and *Xcm* I (NEB ref#R0533S) restriction enzymes in a final mix of 50 mM NaCl, 10 mM Tris-HCl pH 7.9, 10 mM MgCl₂, 1 mM DTT.
After 60 minutes of incubation at 37°C, 2 µl of the mix are analyzed on a TBE agarose gel to check that a complete digestion of the plasmids has occurred.
Then tube is incubated at 80°C for 30 minutes to heat inactivate restriction enzymes.

### Ligation of the linear fragments from the third cleavage condition :

After cooling the tubes on ice, one microliter of T4 DNA ligase (NEB ref #M0202S) is added in each tube with a final ATP concentration of 1mM.
Tube is then incubated at 16°C for one night. *E. coli* DH5α chimio competent cells are then transformed with the resulting ligation products, and plated on LB agar plates containing 10 µg/ml of ampicilline. Plates are incubated overnight at 37°C.

### Selection of the recombinant circular DNA molecules :

Each circular DNA molecule with a shuffled metabolic pathway are recovered from *E*. *coli* DH5-α and these shuffled minimal PKS are cloned in a vector having all the necessary elements for the expression of tetracenomycin C in *S. lividans.* The resulting plasmids are transferred in *Streptomyces* by conjugation to enable the *in vivo* production of these molecules as described in patent WO 2004013327.

Briefly, conjugation experiments are done using 6x10⁶ *E coli* S17.1 cells containing conjugative plasmids or fosmids. The *E coli* cells are grown in LB media with adequat antibiotic. The cells are collected by centrifugation, washed two times using same volume of LB media and concentrated to 10⁸ cells/ml and overlaid on LB plates containing 2x10⁶ pregerminated *Streptomyces lividans* TK24 spores. The cells mixture are grown overnight at 30°C and *E*. *coli* cells are washed three times using 2 ml of LB media. The plates are overlaid using top agar containing NAL (nalidixic acid) and the appropriated antibiotic. Plates are incubated for 4 days at 30°C and transformant *streptomyces* colonies are isolated on HT medium (Pridham et al., 1957, Antibiotics Annual, 1956-1957, 947-953) containing NAL and the same appropriated antibiotic.

Several colonies from each conjugation are isolated and cultivated in liquid Hickey-Tresner (HT) medium, supplemented with beef extract, for six days. Supernatants of each culture are tested on a *Bacillus* sensitivity agar plate (*Bacillus* seeded LB-agar medium plates with wells in which extracts are deposited. After 14-16 hours at 37°C, inhibitory halo appears surrounding the positive anti-infective extracts).

Colonies showing an anti-infective activity are selected, and cultured in larger volumes to obtain higher amounts of the corresponding active compounds. These compounds are tested on a panel of pathogenic strains, and on cancer cell lines, and compared to the activity effect and profile of the parental compound.

In parallel, plasmids from colonies showing an anti-infective activity are prepared and purified with the Quigen Midi Kit, to check their restriction profile as compared to the parental plasmid, and validate that proper recombination events have occurred.

These plasmids can be involved in subsequent rounds of circular DNA molecules cleavage, ligation and selection to further improve the activity profile of these synthesis pathways.

This example demonstrates first that random recombination of secondary metabolites can be achieved through recursive cleavage/ligation of circular DNA molecules, to generate recombined pathways. These recombined pathways can lead to either improved levels of secondary metabolites production, synthesis of molecules with novel activity profiles (active against more pathogens for example), or to compounds with modified activities (better bioavailability, solubility, lower cytotoxicity,...).

Such recombination of entire pathways would have been more difficult, or even impossible through regular DNA shuffling technologies, as these are mainly based on hybridization steps that are physically less efficient for high molecular weight DNA. Moreover, with polymerase based DNA shuffling technologies such as described by Stemmer (1994), additional random points mutations incorporated by the polymerase would dramatically decrease the number of final active pathways, thus lowering the chance to rapidly discover new active compounds.

The method of the present invention enables to create conservative (mutation free) recombination events at very specific points of synthetic pathways. As recombination can be tightly controlled through the selection of proper restriction sites, the operator can design and rationalize its recombinant population to keep the overall structure of the synthesis genes. By choosing both the restriction sites, their combination and their order of use, one can easily obtain selectively several recombination products that may encode for different performing synthesis products.

### EXAMPLE 3 : Shuffling of a metabolic pathway (with no intermediate amplification of recombined libraries)

In this example, we apply the same methodology than in example 2, despite that reaction recombination reactions are successively achieved with no intermediate library amplification by bacterial transformation.

***Construction of the initial circular DNA molecule*** is achieved as described in example 2.

***First cleavage of the circular DNA molecules (shuffling of the first ORF of the cluster)*** is achieved as described in example 2, despite that 800 ng of DNA were used instead of 200 ng.

### Ligation of the linear fragments from the first cleavage condition :

After cooling the tubes on ice, one microliter of T4 DNA ligase (NEB ref #M0202S) is added in each tube with a final ATP concentration of 1 mM.
Tube is then incubated at 16°C for one night. (*nota* : ligation time may be decreased to several hours).
The resulting ligation products are then treated with a 1/1 phenol-chloroform mix, and then ethanol precipitated.

### Second cleavage of the circular DNA molecules (shuffling in the second ORF of the cluster) :

To continue the random recombination process, a second cycle of cleavage and ligation is achieved, by directly resuspending the ethanol precipitated DNA from previous step, and treating it in a final volume of 100 µl and treated with *Xmn* I (NEB ref#R0194S) and *Sph* I (NEB ref#R0182S) restriction enzymes in a final mix of 50 mM potassium acetate, 20 mM Tris-acetate, 10 mM Mg acetate, 1 mM DTT, pH 7.9, 100µg/ml BSA.
After 60 minutes of incubation at 37°C, 2 µl of the mix are analyzed on a TBE agarose gel to check that a complete digestion of the plasmids has occurred.
Then tube is incubated at 80°C for 30 minutes to heat inactivate restriction enzymes.

***Ligation of the linear fragments from the second cleavage condition*** is achieved as for ligation of linear fragments from the first cleavage condition.

### Third cleavage of the circular DNA molecules (shuffling in the third ORF of the cluster):

To continue the random recombination process, a third cycle of cleavage and ligation is achieved, by directly resuspending the ethanol precipitated DNA from previous step, and treating it a final volume of 100 µl and treated with *Xmn* I (NEB ref#R0194S) and *Xcm* I (NEB ref#R0533S) restriction enzymes in a final mix of 50 mM NaCl, 10 mM Tris-HCl pH 7.9, 10 mM MgCl₂, 1 mM DTT.

After 60 minutes of incubation at 37°C, 2 µl of the mix are analyzed on a TBE agarose gel to check that a complete digestion of the plasmids has occurred.
Then tube is incubated at 80°C for 30 minutes to heat inactivate restriction enzymes.

***Ligation of the linear fragments from the third cleavage condition*** is achieved as for ligation of linear fragments from the third cleavage condition described in example 2.

***Selection of the recombinant circular DNA molecules*** is achieved as described in example 2.

This implementation of the method enables to create more rapidly recombined library (the limiting time is the ligation time, which can be reduced to few minutes by using specific ligation kits such as the Quick Ligation^{™} kit from New England Biolabs). It also avoids, if relevant in an experiment, to introduce a statistical bias through transformation of ligation products at each cleavage/ligation step.

## Claims

1. A method for *in vitro* recombining polynucleotides sequences of interest from at least two initial different polynucleotide sequences of interest by cleavage/ligation of circular DNA molecules, comprising:
1) providing at least two circular DNA molecules, each comprising a vector molecule and one of the at least two different polynucleotide sequences of interest;
2) causing the cleavage of said circular DNA molecules at a unique cleavage site into the vector molecule and at a unique cleavage site into the polynucleotide sequences of interest, thereby releasing linear cleavage fragments;
3) ligating said linear cleavage fragments to reassemble circular DNA molecules, thereby some of said resassembled circular DNA molecules have a recombined polynucleotide sequences of interest; and,
4) optionally, repeating steps (2) and (3) using other unique cleavage sites into the polynucleotide sequences of interest.

2. Method according to claim 1, wherein step (1) comprises introducing the at least two different polynucleotide sequences of interest into the vector molecule so as to obtain at least two circular DNA molecules, each comprising one of the at least two different polynucleotide sequences of interest.

3. Method according to claim 1 or 2, wherein the cleavage of said circular DNA molecules is performed by endonuclease(s) specific of a unique endonuclease cleavage site into the vector molecule and endonuclease(s) specific of a unique endonuclease cleavage site into the polynucleotide sequences of interest.

4. Method according to claim 1 or 2, wherein the cleavage of said circular DNA molecules is performed by a step of PCR amplification of two fragments covering together said circular DNA molecules, and each fragment comprising a part of the polynucleotide sequence of interest and a part of the vector molecule.

5. Method according to claim 4, wherein each primer used in the step of PCR amplification comprises a unique endonuclease cleavage site, and the step of PCR amplification is followed by a step of cleavage by said endonuclease.

6. Method according to claim 1 or 2, wherein the step (2) comprises the following steps : a) cleaving one of the at least two circular DNA molecules by endonuclease(s) at a first unique endonuclease cleavage site into the vector molecule and at a second unique endonuclease cleavage site into the polynucleotide sequences of interest, b) cleaving the other of the at least two circular DNA molecules by endonuclease(s) at the first unique endonuclease cleavage site into the vector molecule and at a third unique endonuclease cleavage site into the polynucleotide sequences of interest, c) treating the releasing linear cleavage fragments by a 3' exonuclease (3' ends trimming), and d) eliminating the 5' ovelapping single stranded ends.

7. Method according to any one of the preceding claims, wherein the method comprises an additional step of selecting recombined polynucleotide sequences of interest having an improved characteristic as compared to a reference polynucleotide sequence of interest.

8. Method according to any one of the preceding claims, wherein the polynucleotide sequences of interest are derived from genomic, metagenomic, environmental, plasmidic, mitochondrial or any complex DNA of plants, animals, insects, bacteria, cells, virus, phage or any existing organism.

9. Method according to any one of the preceding claims, wherein the initial polynucleotide sequences of interest are generated by prior steps of DNA recombination, mutagenesis or synthesis.

10. Method according to any one of the preceding claims, wherein the vector molecule comprises at least one replication origin and at least one selection marker.

11. Method according to claim 10, wherein the vector molecule is cleaved into the at least one selection marker.

12. Method according to claim 3 or 5, wherein the endonucleases used to cleave the polynucleotide sequences of interest generate compatible ends for the ligation step (3), thereby allowing recombination to occur between polynucleotide sequences of interest.

13. Method according to claim 12, wherein the same endonuclease is used to cleave all the polynucleotide sequences of interest.

14. Method according to claim 12 or 13, wherein the endonucleases generate overhanging ends into the polynucleotide sequences of interest.

15. Method according to any one of claims 3, 5, 6 and 13-14, wherein the endonuclease used to cleave the vector molecule generates blunt ends or overhanging ends which are not compatible with the ends into the polynucleotide sequences of interest for the ligation step (3).

16. Method according to any one of the preceding claims, wherein the ligating step is performed with a phage T4 DNA ligase or a thermostable ligase.

17. Method according to any one of the preceding claims, wherein the step (2) of cleavage for each circular DNA molecule comprising one different polynucleotide sequence of interest is performed separately, and the step (3) of ligation is performed with a defined mixture of at least two products of individual cleavage of circular DNA molecules.

18. Method according to any one of claims 1-16, wherein the steps (2) and (3) are performed with a pool of circular DNA molecules.

19. Method according to any one of the preceding claims, wherein said polynucleotide sequences of interest encode a protein, preferably an enzyme, and the method allows to shuffle protein domains, catalytic sites, affinity sites or any combination thereof.

20. Method according to any one of claims 1-18, wherein said polynucleotide sequences of interest are metabolite(s) production pathway or part thereof.

21. Method according any one of the preceding claims, wherein the method is performed by an automated device, the method comprising :
i) feeding the automated device with at least two circular DNA molecules, some cleavage reagents and some ligation reagents;
ii) running cleavage reaction(s);
iii) running ligation reaction(s); and
iv) optionally, repeating steps (ii) and (iii) with other cleavage reagents.

22. Method according any one of the preceding claims, wherein the method is performed by a software automated device, the method comprising a previous step of determining by a software a sorted list of cleavage reagents.


SEQUENCE LISTING

<110>  LIBRAGEN

<120>  Method of in vitro polynucleotide sequences shuffling by recursive
circular DNA molecules fragmentation and ligation

<130>  B0460EP

<140>  EP 06290534.4

<141>  2006-04-04

<160>  1

<170>  PatentIn version 3.1

<210>  1

<211>  2831

<212>  DNA

<213>  S. glaucescens

<400>  1
atgacccggc acgcggagaa gcgggtggtg atcaccggca tcggtgtgcg agccccggga      60

ggcgccggaa ccgcggcctt ctgggacctg ctcaccgccg gacgcaccgc caccaggacg     120

atctccctgt tcgacgccgc gccctaccgg tcccggatag ccggagagat cgacttcgac     180

ccgatcggcg agggcctgtc gccccggcag gcgtcgacgt acgaccgggc cacgcagctc     240

gccgtcgtct gcgcccgcga ggcgctgaag gacagcgggc tggacccggc cgcggtgaac     300

ccggaacgga tcggggtgag catcggcacc gccgtcggct gcaccaccgg cctcgaccgc     360

gagtacgcgc gggtcagcga gggcggctcg cgctggctgg tcgaccacac gctcgcggtg     420

gagcagctct tcgactactt cgtcccgacc tccatctgcc gggaggtcgc ctgggaggcg     480

ggggccgagg gaccggtgac ggtggtgtcg accgggtgca cctcgggcct cgacgccgtc     540

ggatacggca cggagctcat ccgggacggg cgcgccgacg tggtcgtctg cggcgccacc     600

gacgcgccga tctcccccat caccgtggcc tgcttcgacg ccatcaaggc gacgtcggcc     660

aacaacgacg acccggccca cgcctcccgc cccttcgacc gcaaccgcga cggcttcgtg     720

ctcggggagg gctcggcggt cttcgtcctg gaggagctgt ccgccgcccg ccggcgagga     780

gcgcacgcct acgcggaggt ccgcggcttc gccacacggt cgaacgcctt ccacatgacc     840

ggtctgaagc cggacggcag ggagatggcg gaggccatca ccgccgccct ggaccaggcc 900
cgccgcaccg gcgacgacct gcactacatc aacgcgcacg gctccggcac ccggcagaac 960
gaccggcacg agacggcggc cttcaagcgc agcctggggc aacgggcgta cgacgtgccc 1020
gtcagctcca tcaagtccat gatcggccac tcgctcggag cgatcggctc cctcgaactg 1080
gccgcctgcg cgctggcgat cgaacacggc gtgatcccgc ccaccgccaa ctacgaggaa 1140
cccgaccccg aatgcgacct cgactacgtg ccgaacgtcg cgcgggaaca gcgggtcgac 1200
accgtgctca gcgtcggcag cggattcggc ggcttccaga gcgccgcggt gctggcccgg 1260
ccgaaggaga cccgctcatg agcgcgccgg cccccgtcgt ggtcaccggc ctgggcatcg 1320
tggcacccaa cggcaccggc accgaggagt actgggcggc cacgctcgcc ggcaagagcg 1380
gcatcgacgt catccagcgg ttcgaccccc acggctaccc cgtccgggtg ggaggcgagg 1440
tgctggcctt cgacgccgcc gcccatctcc ccgggcgcct gctgccgcag accgaccgca 1500
tgacccagca cgccctggtg gccgccgaat gggccctggc cgacgccggg ctggagcccg 1560
agaagcagga cgagtacggc ctcggcgtcc tgacggccgc cggcgcggga gggttcgagt 1620
tcggccagcg cgagatgcag aaactctggg gaacgggccc ggaacgcgtc agcgcctacc 1680
agtcgttcgc ctggttctac gcggtgaaca ccggccagat ctccatccgc cacggcatgc 1740
gcgggcacag ctccgtgttc gtcaccgagc aggccggcgg tctcgacgcc gccgcccacg 1800
cggcccgcct gctgcgcaag gggaccctga acaccgccct gaccggcgga tgcgaagcct 1860
ccctctgccc ctggggcctg gtcgcccaga tcccgagcgg cttcctgagc gaggccaccg 1920
acccgcacga cgcctacctg cccttcgacg cgcgggcggc cgggtacgtg cccggcgagg 1980
gcggagccat gctggtcgcc gagcgggcgg actccgcacg cgaacgggac gccgccacgg 2040
tctacgggag gatcgccggg cacgcctcca ccttcgacgc ccgccccggc accggccggc 2100
ccaccgggcc ggcccgggcc atccgcctgg ccctcgaaga ggcccgggtc gcgcccgagg 2160
acgtcgacgt ggtctacgcc gacgccgctg gagtgccggc cctcgaccgc gcggaggcgg 2220
aggccctcgc cgaggtcttc gggcccgggg ccgtaccggt caccgcgccc aagacgatga 2280
ccggacggct ctacgccggg ggagcggccc tcgacgtcgc gaccgcgctg ctgtccatca 2340
gggactgtgt cgtcccgccg accgtcggca ccggtgcgcc cgcgccgggg ctcggcatcg 2400
acctggtgct ccaccagccc cgtgagctgc gggtggacac cgccctggtc gtcgcgcggg 2460
gcatgggcgg attcaactcc gccctggtcg tccggcgcca cggctgaccg agccgccgcc 2520
cccgctctcc gcgcccgagc gcgcatcacc tcatcacacc actcatccgg aggttcaccg 2580
tgccccagat cggcctgccc cgactcgtcg agatcatcag ggaatgcgca ggagaccccg 2640
acgaacggga cctggacggc gacatcctcg acgtcaccta ccaggacctc ggctacgact 2700
ccatcgccct gctggagatc tccgcgaagc tggaacagga cctcggggtg tcgataccgg 2760
gggaggagct gaagaccccc cggcacaccc tccacctcgt caacaccgag acggccgggg 2820
aggtcgcctg a 2831